# EUROPEAN PATENT SPECIFICATION

(11) **EP 1 824 970 B1**
(45) Date of publication and mention of the grant of the patent: **10.08.2011**
(21) Application number: 05816997.0
(22) Date of filing: 07.10.2005
(51) Int. Cl.: C12N 9/34, C12N 15/56, C12P 19/20, C12P 7/06

(54) **TRICHODERMA REESEI GLUCOAMYLASE AND HOMOLOGS THEREOF**
TRICHODERMA REESEI-GLUCOAMYLASE UND HOMOLOGE DAVON
TRICHODERMA REESEI GLUCOAMYLASE ET SES HOMOLOGUES

(30) Priority: 30.11.2004 WO PCT/US2004/040040; 09.12.2004 WO PCT/US2004/041276; 28.01.2005 US 647925 P; 24.05.2005 WO PCT/US2005/018212; 24.05.2005 WO PCT/US2005/018214
(43) Date of publication of application: 29.08.2007
(73) Proprietor: GENENCOR INTERNATIONAL, INC., Palo Alto, California 94304 (US)
(72) Inventor: DUNN-COLEMAN, Nigel, Genencor International, Inc., Palo Alto, California 94304 (US); NEEFE-KRUITHOF, P., Genencor International Inc., Palo Alto, California 94304 (US); PILGRIM, Craig E., Genencor International, Inc., Palo Alto, California 94304 (US); WARD, Donald E., Genencor International, Inc., Palo Alto, California 94304 (US); VAN SOLINGEN, Pieter, Genencor International, Inc., Palo Alto, California 94304 (US)
(74) Representative: Kiddle, Simon John
(86) International application number: PCT/US2005/036307
(87) International publication number: WO 2006/060062

(56) References cited:
- WO-A-02/46429
- WO-A-03/068976
- WO-A1-2004/113551
- WO-A1-2004/113551
- WO-A2-2004/080923
- WO-A2-2004/080923
- US-A- 5 665 585
- FAGERSTROM R: "Purification and specificity of recombinant Hormoconis resinae glucoamylase P and endogenous glucoamylase from Trichoderma reesei" ENZYME AND MICROBIAL TECHNOLOGY, STONEHAM, MA, US, vol. 16, no. 1, 1994, pages 36-42, XP002325130 ISSN: 0141-0229
- FAGERSTRÖM R ET AL: "Characterization, subsite mapping and partial amino acid sequence of glucoamylase from the filamentous fungus Trichoderma reesei." BIOTECHNOLOGY AND APPLIED BIOCHEMISTRY RES. LAB., ALKO LTD., POB 350, SF-00101 HELSINKI, FINLAND, vol. 21, no. 2, 1995, page 223, XP009063876
- CHAMBERGO FELIPE S ET AL: "Elucidation of the metabolic fate of glucose in the filamentous fungus Trichoderma reesei using expressed sequence tag (EST) analysis and cDNA microarrays." THE JOURNAL OF BIOLOGICAL CHEMISTRY. 19 APR 2002, vol. 277, no. 16, 19 April 2002 (2002-04-19), pages 13983-13988, XP002373840 ISSN: 0021-9258
- DATABASE EMBL [Online] 6 February 2002 (2002-02-06), "TrEST-A4050 TrEST-A Hypocrea jecorina cDNA clone Tr-A4050 5' similar to glucoamylase, mRNA sequence." XP002374132 retrieved from EBI accession no. EM_PRO:BM076396 Database accession no. BM076396
- DATABASE EMBL [Online] 20 November 2003 (2003-11-20), "TrEST-A0916 TrEST-A Hypocrea jecorina cDNA clone Tr-A0916 5' similar to glucan 1,4-alpha-glucosidase (EC 3.2.1.3) precursor - Neurospora crassa, mRNA sequence." XP002374133 retrieved from EBI accession no. EM_PRO:CF943925 Database accession no. CF943925
- DATABASE EMBL [Online] 20 November 2003 (2003-11-20), "TrEST-A2623 TrEST-A Hypocrea jecorina cDNA clone Tr-A2623 5' similar to glucan 1,4-alpha-glucosidase (EC 3.2.1.3) precursor - Neurospora crassa, mRNA sequence." XP002374134 retrieved from EBI accession no. EM_PRO:CF944284 Database accession no. CF944284
- DATABASE EMBL [Online] 20 November 2003 (2003-11-20), "TrEST-A0518 TrEST-A Hypocrea jecorina cDNA clone Tr-A0518 5' similar to glucan 1,4-alpha-glucosidase (EC 3.2.1.3) precursor - Neurospora crassa, mRNA sequence." XP002374135 retrieved from EBI accession no. EM_PRO:CF944444 Database accession no. CF944444
- DATABASE EMBL [Online] 26 April 2003 (2003-04-26), "tric021xg09 T.reesei mycelial culture, Version 3 april Hypocrea jecorina cDNA clone tric021xg09, mRNA sequence." XP002374136 retrieved from EBI accession no. EM_PRO:CB900226 Database accession no. CB900226
- STONE P J ET AL: "CLONING AND SEQUENCE ANALYSIS OF THE GLUCOAMYLASE GENE OF NEUROSPORA CRASSA" CURRENT GENETICS, NEW YORK, NY, US, vol. 24, no. 3, 1993, pages 205-211, XP009005941 ISSN: 0172-8083
- BARNETT ET AL: "Cloning and amplification of the gene encoding an extracellular beta-glucosidase from Trichoderma reesei: Evidence for improved rates of saccharification of cellulosic substrates" BIO/TECHNOLOGY, NATURE PUBLISHING CO. NEW YORK, US, vol. 9, no. 9, June 1991 (1991-06), pages 562-567, XP002109025 ISSN: 0733-222X
- TAKASHIMA S ET AL: "Molecular cloning and expression of the novel fungal beta-glucosidase from H.grisea and T.reesei" JOURNAL OF BIOCHEMISTRY, JAPANESE BIOCHEMICAL SOCIETY / OUP, TOKYO, JP, vol. 125, no. 4, January 1999 (1999-01), pages 728-736, XP002964031 ISSN: 0021-924X
- MORIMURA S ET AL: "GENETIC ENGINEERING OF WHITE SHOCHU-KOJI TO ACHIEVE HIGHER LEVELS OF ACID-STABLE ALPHA-AMYLASE AND GLUCOAMYLASE AND OTHER PROPERTIES WHEN USED FOR SHOCHU MAKING ON A LABORATORY SCALE", JOURNAL OF THE INSTITUTE OF BREWING, INSTITUTE OF BREWING. LONDON, GB, vol. 105, no. 5, 1 September 1999 (1999-09-01), pages 309-314, XP001205897, ISSN: 0046-9750
- KANEKO AKIHIRO ET AL: "Molecular cloning and determination of the nucleotide sequence of a gene encoding an acids-stable alpha-amylase from Aspergillus kawachii", JOURNAL OF FERMENTATION AND BIOENGINEERING, SOCIETY OF FERMENTATION TECHNOLOGY, JP, vol. 81, no. 4, 1 January 1996 (1996-01-01), pages 292-298, XP002256177, ISSN: 0922-338X
- MORIMURA S ET AL: "GENETIC ENGINEERING OF WHITE SHOCHU-KOJI TO ACHIEVE HIGHER LEVELS OF ACID-STABLE ALPHA-AMYLASE AND GLUCOAMYLASE AND OTHER PROPERTIES WHEN USED FOR SHOCHU MAKING ON A LABORATORY SCALE" JOURNAL OF THE INSTITUTE OF BREWING, INSTITUTE OF BREWING. LONDON, GB, vol. 105, no. 5, 1 September 1999 (1999-09-01), pages 309-314, XP001205897 ISSN: 0046-9750
- KANEKO AKIHIRO ET AL: "Molecular cloning and determination of the nucleotide sequence of a gene encoding an acids-stable alpha-amylase from Aspergillus kawachii" JOURNAL OF FERMENTATION AND BIOENGINEERING, SOCIETY OF FERMENTATION TECHNOLOGY, JP, vol. 81, no. 4, 1 January 1996 (1996-01-01), pages 292-298, XP002256177 ISSN: 0922-338X

## Description

### FIELD OF THE INVENTION

The present invention relates to new enzyme compositions comprising a glucoamylase and an alpha amylase useful for the production of glucose and other end products from starch. The enzyme compositions are suitable for use in various processes and are particularly suitable for use under conditions of conventional high temperature starch processing and under conditions of non-cook or low temperature starch processing.

### BACKGROUND OF THE INVENTION

Glucoamylase enzymes (α-1,4-glucan glucohydrolases, E.C.3.2.1.3.) are starch hydrolyzing exo-acting carbohydrases. Glucoamylases catalyze the removal of successive glucose units from the non-reducing ends of starch or related oligo and polysaccharide molecules and can hydrolyze both linear and branched glucosidic linkages of starch (amylose and amylopectin).

Glucoamylases are produced by numerous strains of bacteria, fungi, yeast and plants. Particularly interesting glucoamylases are fungal enzymes that are extracellularly produced, for example from strains of *Aspergillus* (Boel et al., (1984) EMBO J. 3:1097-1102; Hayashida et al (1989) Agric. Biol. Chem. 53:923 - 929; USP 5,024,941; USP 4,794,175; and WO 88/09795), *Talaromyces* (USP 4,247,637; USP 6,255,084 and USP 6,620,924), *Rhizopus* (Ashikari et al. (1986) Agric. Biol. Chem. 50:957 - 964; Ashikari et al. (1989) App. Microbiol. and Biotech. 32:129 - 133 and USP 4,863,864), *Humicola* (WO05/052148 and USP 4,618,579) and *Mucor* (Houghton-Larsen et al., (2003) Appl. Microbiol. Biotechnol., 62: 210- 217). Many of the genes, which code for these enzymes have been cloned and expressed in yeast and fungal cells.

Commercially glucoamylases are very important enzymes that have been used in a wide variety of applications requiring the hydrolysis of starch. Glucoamylases are used for the hydrolysis of starch to produce high fructose corn sweeteners, and corn sweeteners comprise over 50% of the US sweetener market. In general, starch hydrolyzing processes involve the use of alpha amylases to hydrolyze the starch to dextrins and glucoamylases to hydrolyze the dextrins to glucose. The glucose is then converted to fructose by other enzymes such as glucose isomerases. Glucose produced by glucoamylases can also be crystallized or used in fermentations to produce other end-products, such as citric acid, ascorbic acid, glutamic acid, 1, 3 propanediol and others. Glucoamylases are used in alcohol production, such as beer production and sake production. Glucoamylases also find use in the production of ethanol for fuel and for consumption. Recently, glucoamylases have been used in low-temperature processes for the hydrolysis of granular (non-cooked) starch. Glucoamylases are also used in the preparation of animal feeds as feed additives or as liquid feed components for livestock animals.

Although glucoamylases have been used successfully for many years, a need still exists for new useful glucoamylases. The present invention is based upon the finding of novel enzyme compositions suitable for use in various applications and particularly starch conversion processes.

### SUMMARY OF THE INVENTION

The present invention provides enzyme compositions, and methods of using said enzyme compositions, as defined in the claims.

The invention relates to an enzyme having glucoamylase activity comprising the amino acid sequence of SEQ ID NO: 4 or substantially homologous sequences thereto and allelic variants and biologically functional fragments thereof.

In another embodiment, the invention is directed to starch conversion processes using the enzyme preparations of the invention. In some embodiments, the composition may be used in a process of converting starch or partially hydrolyzed starch into a syrup containing dextrose. The composition may be used in a process for producing specialty syrups. In further embodiments, the composition may be used in a fermentation to produce end products, such as alcohols and particularly ethanol.

### BRIEF DESCRIPTION OF THE DRAWINGS

FIG.1A - B shows the genomic DNA sequence (SEQ ID NO: 1) coding for the *Trichoderma reesei* glucoamylase of Figure 3.

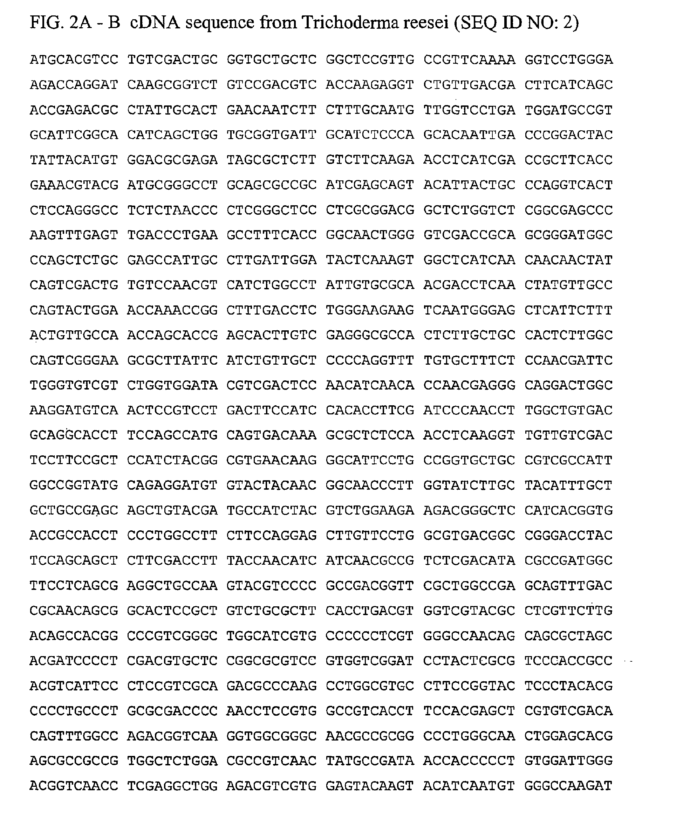

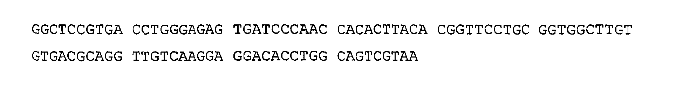

FIG. 2A - B shows the intronless DNA sequence (SEQ ID NO: 2) coding for the *Trichoderma reesei* glucoamylase of Figure 3.
FIG. 3A shows the deduced amino acid sequence (SEQ ID NO: 3) of the *Trichoderma reesei* glucoamylase having 632 amino acids, wherein
   the signal sequence (SEQ ID NO: 38) is in bold and is represented by residue positions 1 - 20;
   the prosequence (SEQ ID NO: 39) is in bold and underlined and represented by residue positions 21 - 33;
   the catalytic domain (SEQ ID NO: 40) is represented by residue positions 34 - 486;
   the linker region (SEQ ID NO: 41) is in italics and represented by residue positions 487 - 523; and
   the starch binding domain (SEQ ID NO: 42) is in italics and underlined and represented by residue positions 524 - 632.
   The N- terminal amino acid residue of the mature protein represented by residue position 34 is serine.

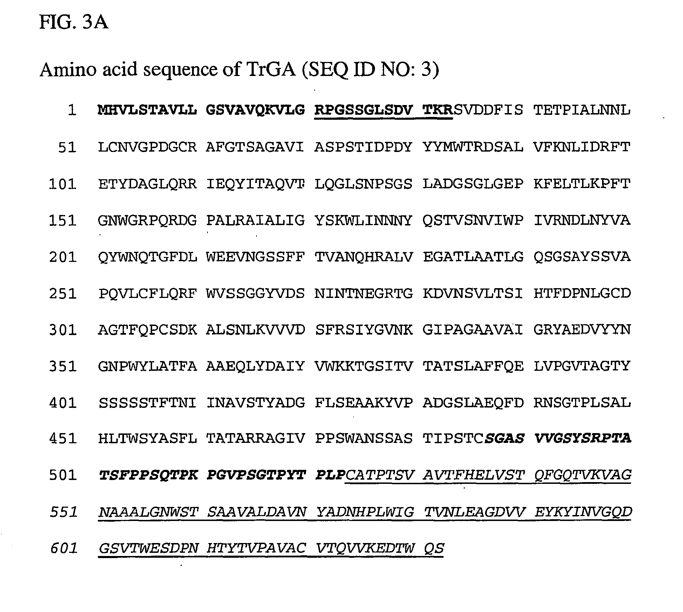

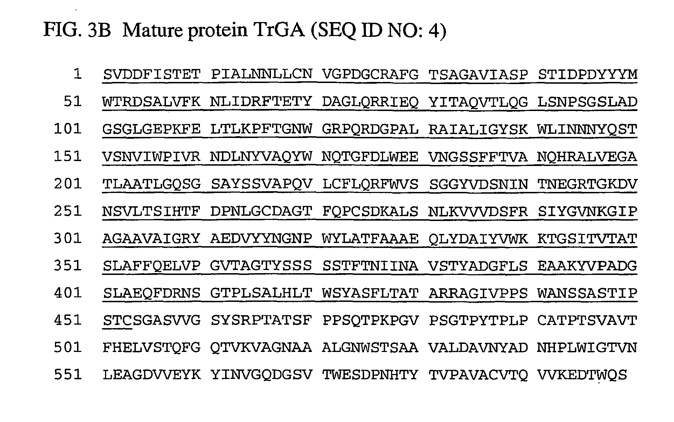

FIG. 3B shows the deduced mature protein sequence (SEQ ID NO: 4) of the *Trichodenna reesei* glucoamylase of Fig. 3A. The mature protein sequence includes the catalytic domain, which is underlined (SEQ ID NO: 40), the linker region (SEQ ID NO: 41) and starch binding domain (SEQ ID NO: 42).

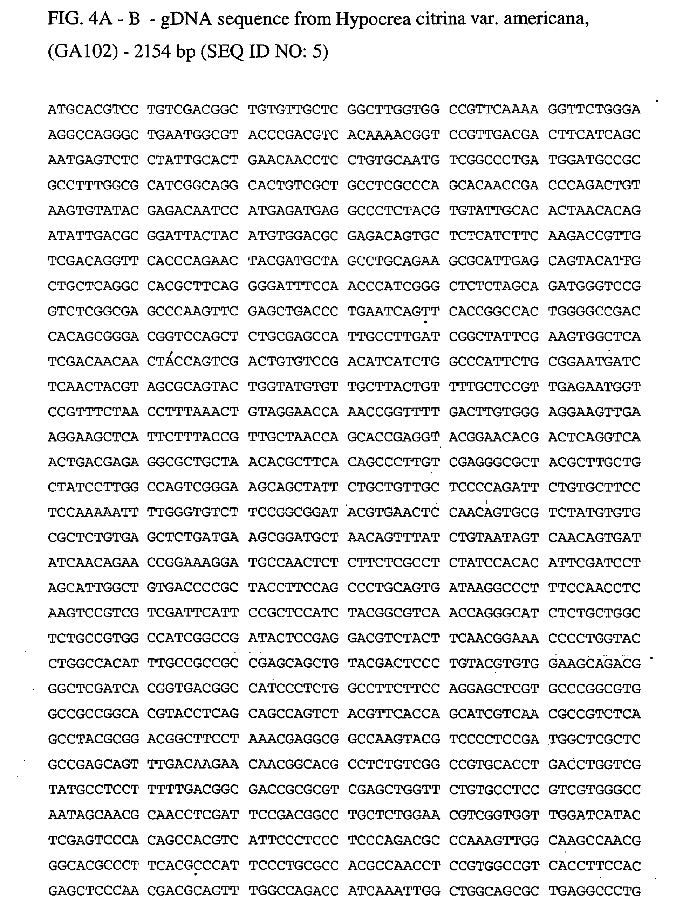

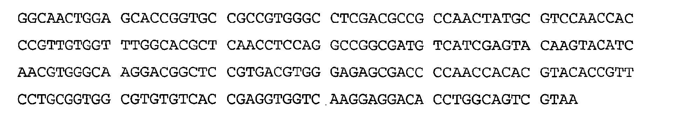

FIG. 4 shows the genomic DNA sequence having 2154 bp (SEQ ID NO: 5) coding for the *Hypocrea citrina var. americana* glucoamylase (GA102) (SEQ ID. NO: 6).

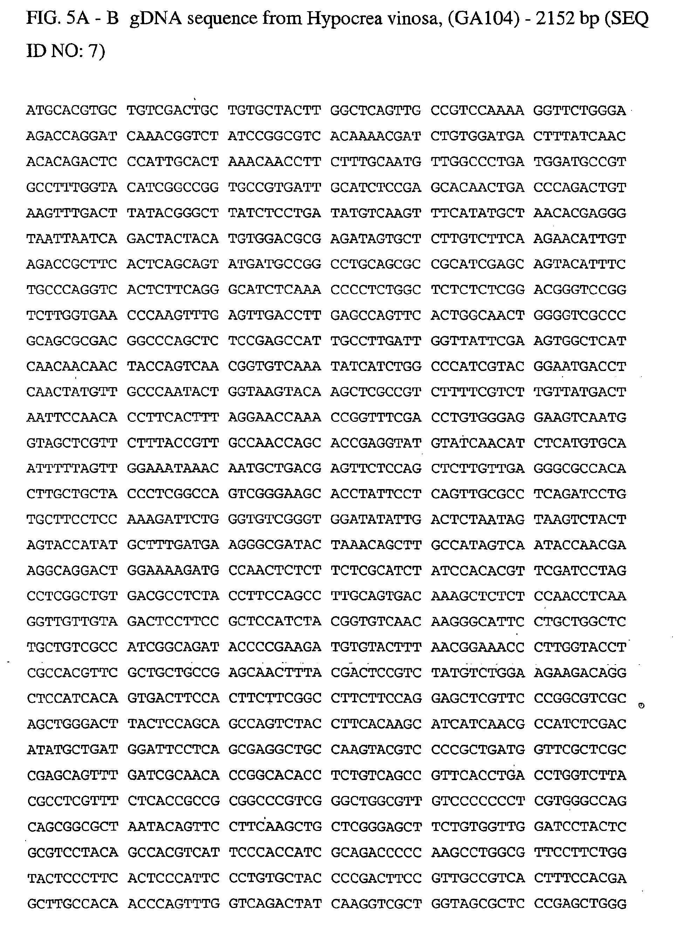

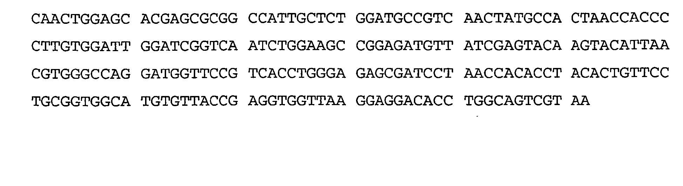

FIG. 5 shows the genomic DNA sequence having 2152 bp (SEQ ID NO: 7) coding for the *Hypocrea vinosa* glucoamylase (GA104) (SEQ ID NO: 8).

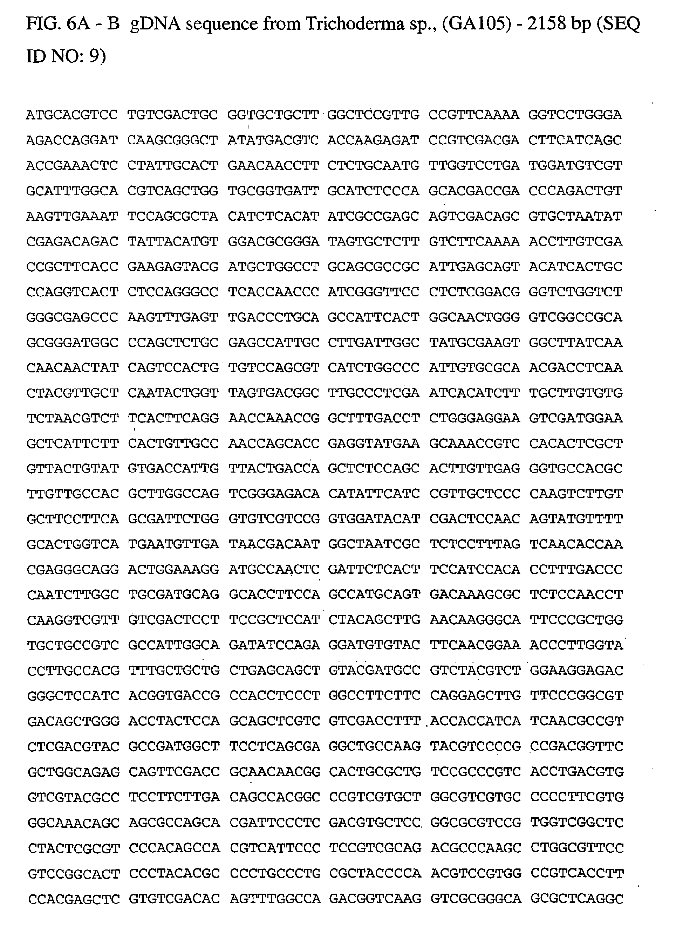

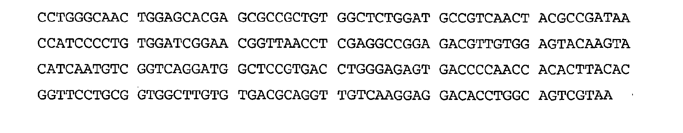

FIG. 6 shows the genomic DNA sequence having 2158 bp (SEQ ID NO: 9) coding for a *Trichoderma* sp. glucoamylase (GA105) (SEQ ID NO: 10).

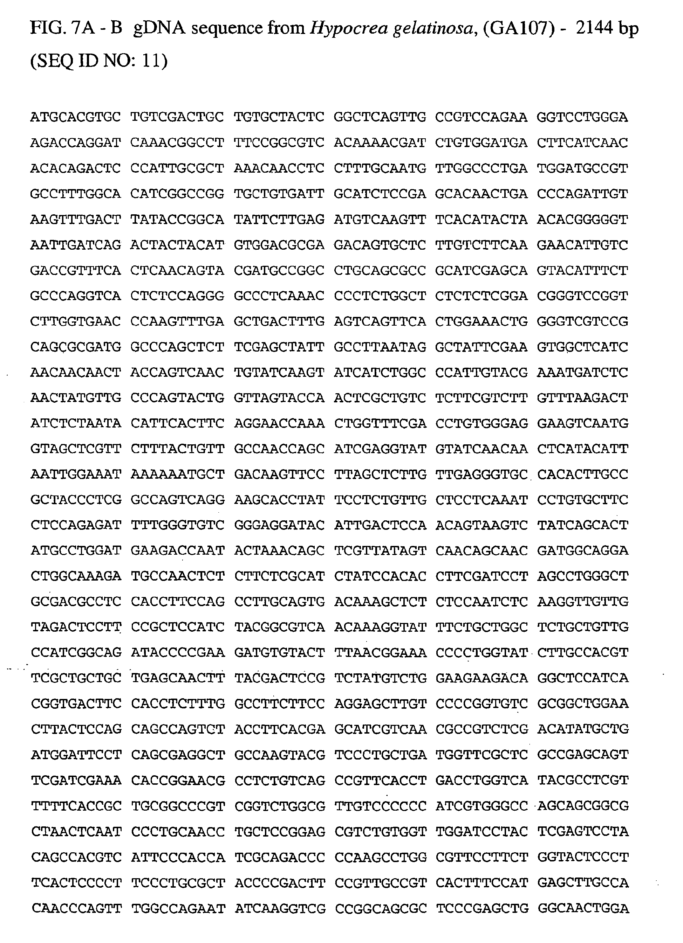

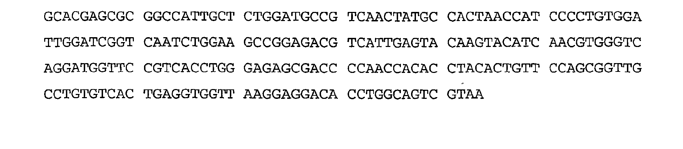

FIG. 7 shows the genomic DNA sequence having 2144 bp (SEQ ID NO: 11) coding for a *Hypocrea gelatinosa* glucoamylase (GA107) (SEQ ID NO: 12).

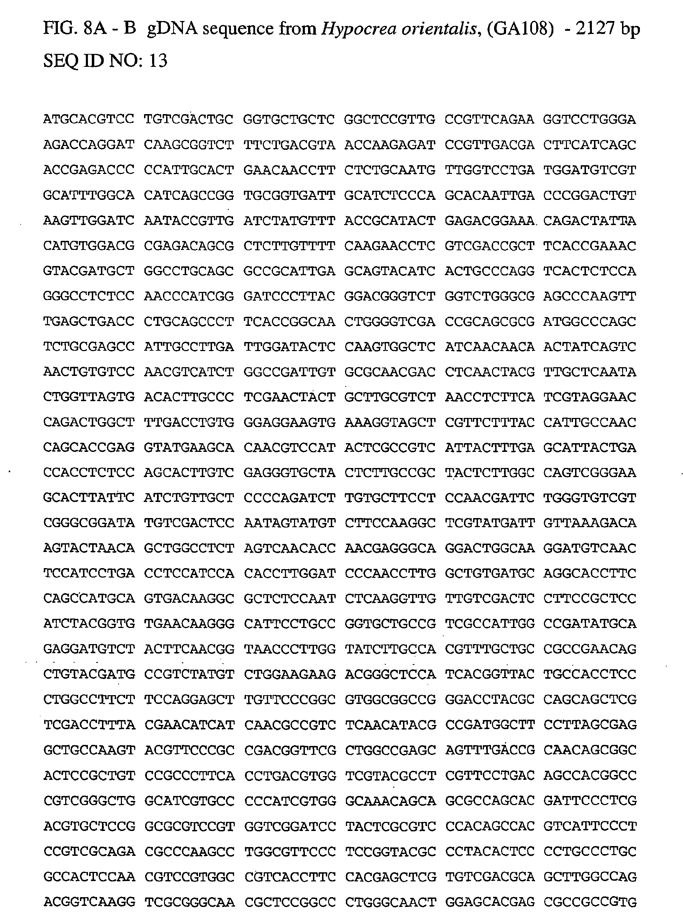

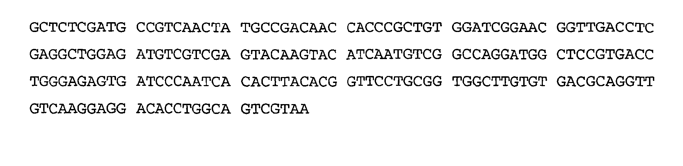

FIG. 8 shows the genomic DNA sequence having 2127 bp (SEQ ID NO: 13) coding for a *Hypocrea orientalis* glucoamylase (GA108) (SEQ ID NO: 14).

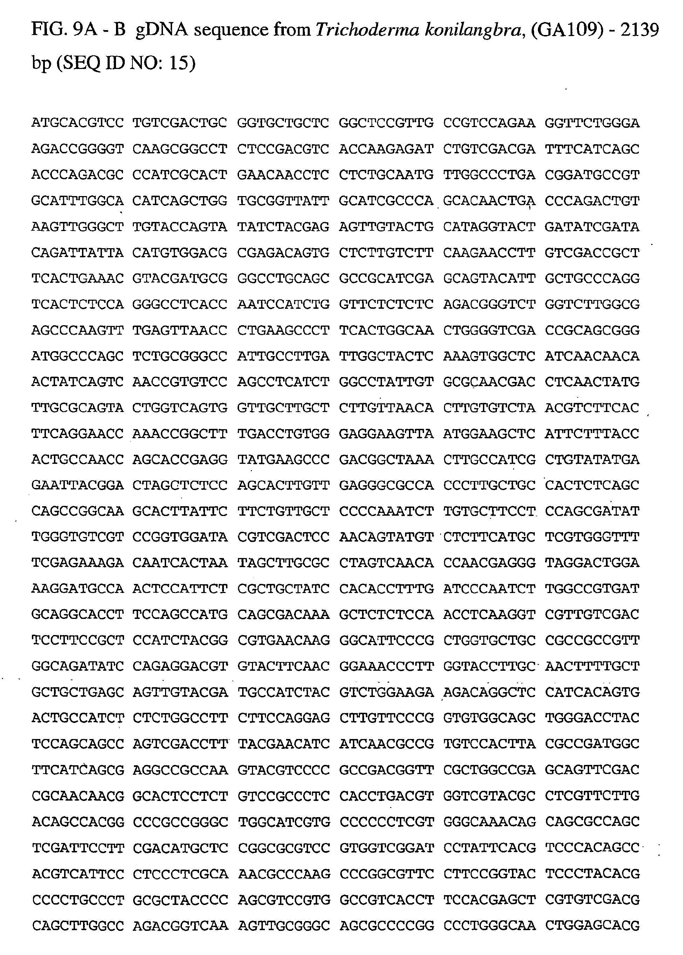

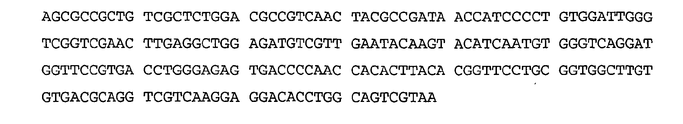

FIG 9 shows the genomic DNA sequence having 2139 bp (SEQ ID NO: 15) coding for a *Trichoderma konilangbra* glucoamylase (GA109) (SEQ ID NO: 16).

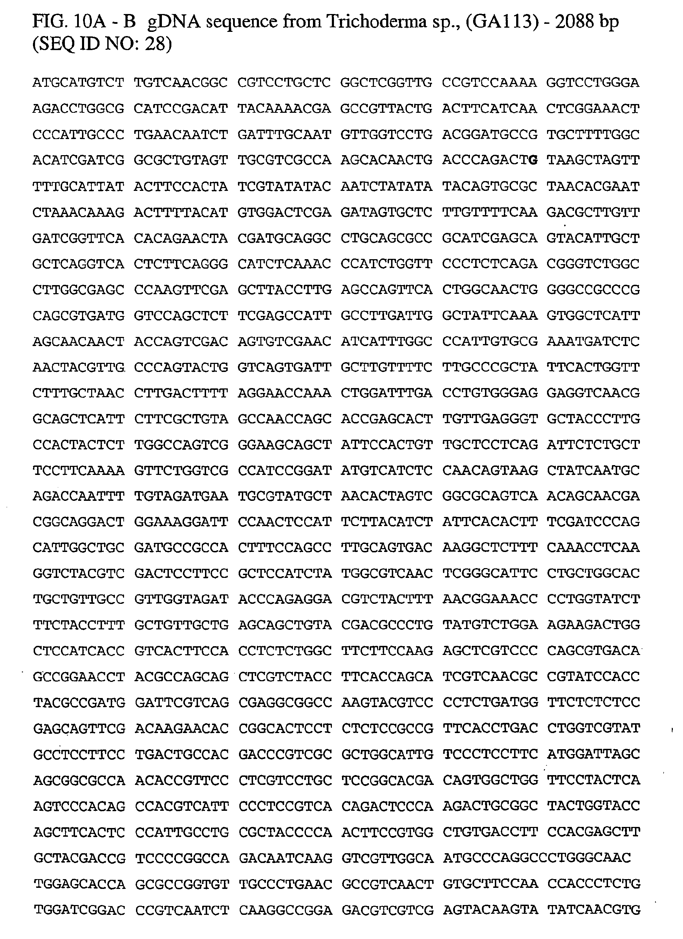

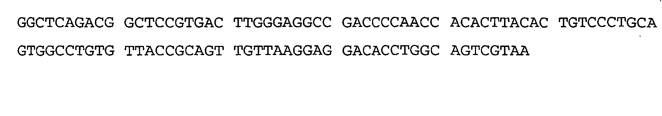

FIG. 10 shows the genomic DNA sequence having 2088 bp (SEQ ID NO: 28) coding for *Trichoderma sp.* glucoamylase (GA113) (SEQ ID NO: 29).

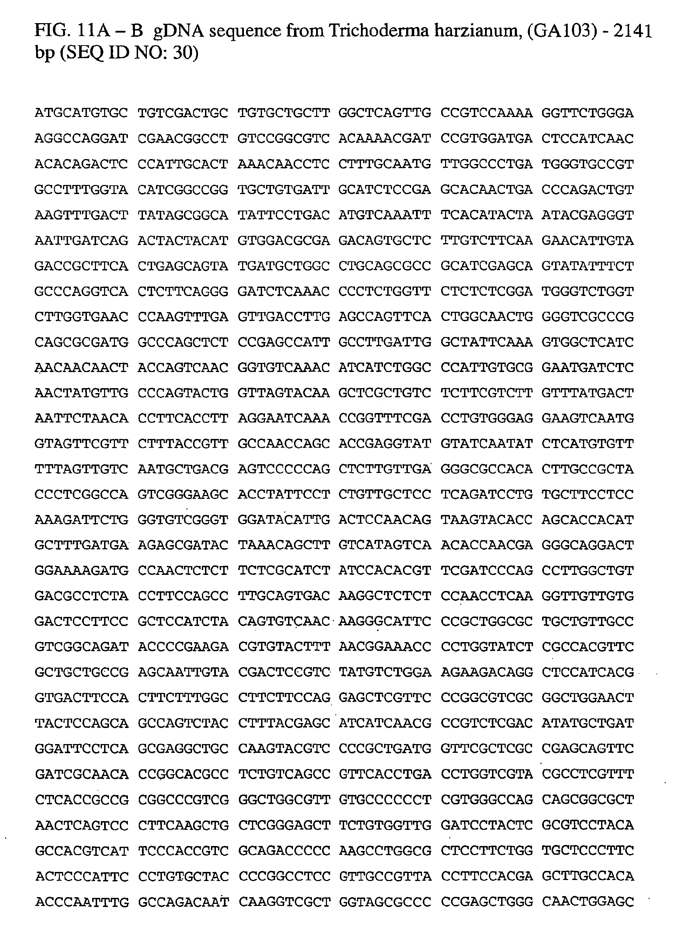

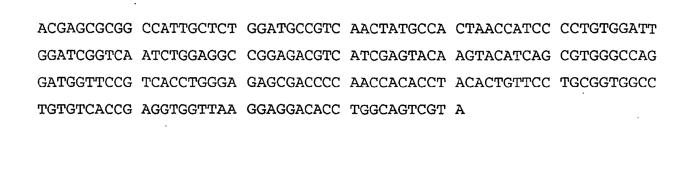

FIG. 11 shows the genomic DNA sequence having 2141 bp (SEQ ID NO: 30) coding for a *Trichoderma harzianum* glucoamylase (GA103) (SEQ ID NO: 31).

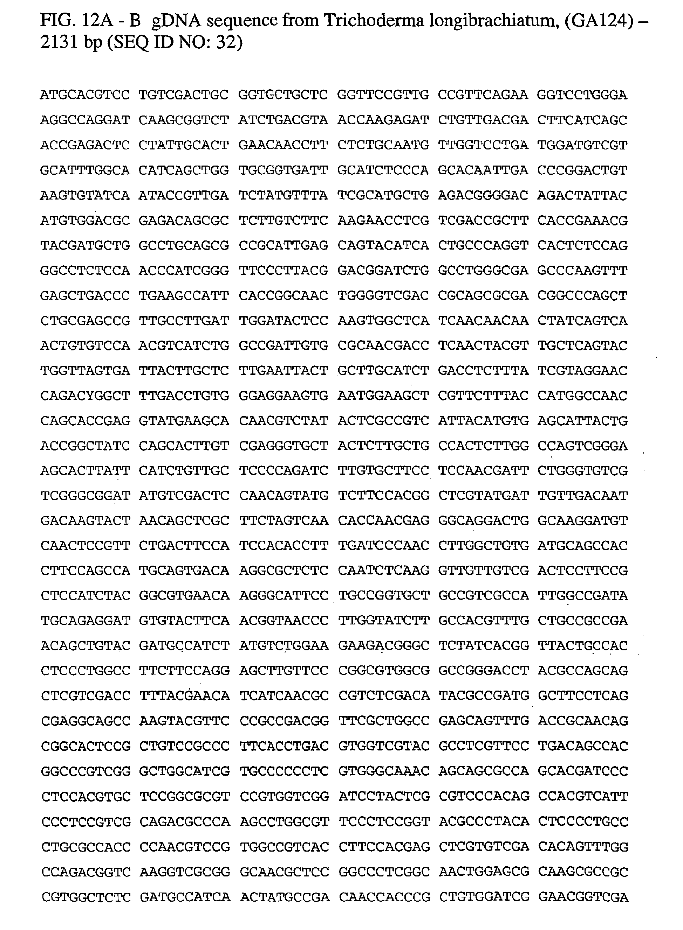

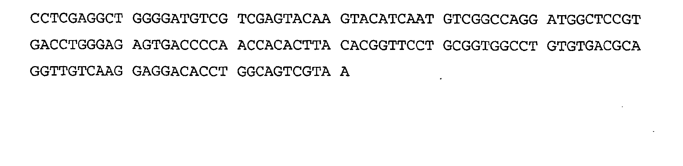

FIG. 12 shows the genomic DNA sequence having 2131 bp (SEQ ID NO: 32) coding for a *Trichoderma longibrachiatum* glucoamylase (GA124) (SEQ ID NO: 33).

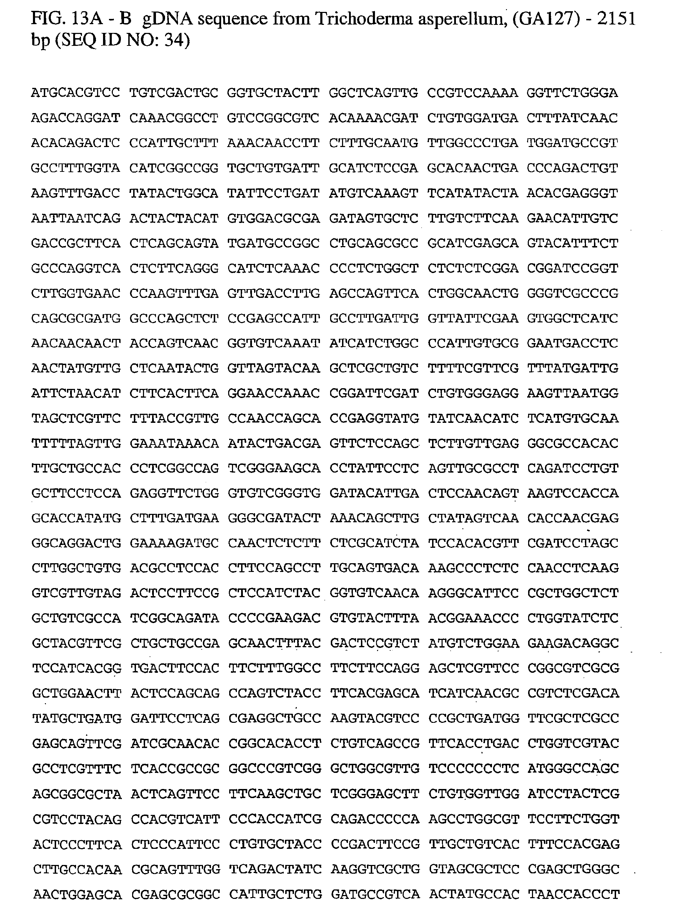

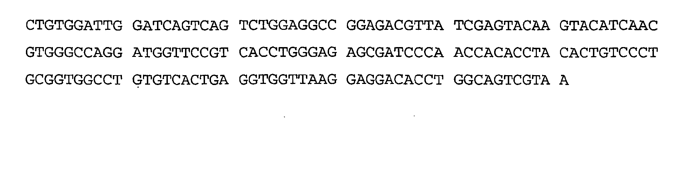

FIG. 13 shows the genomic DNA sequence having 2151bp (SEQ ID NO: 34) coding for *Trichoderma asperellum* glucoamylase (GA127) (SEQ ID NO: 35).

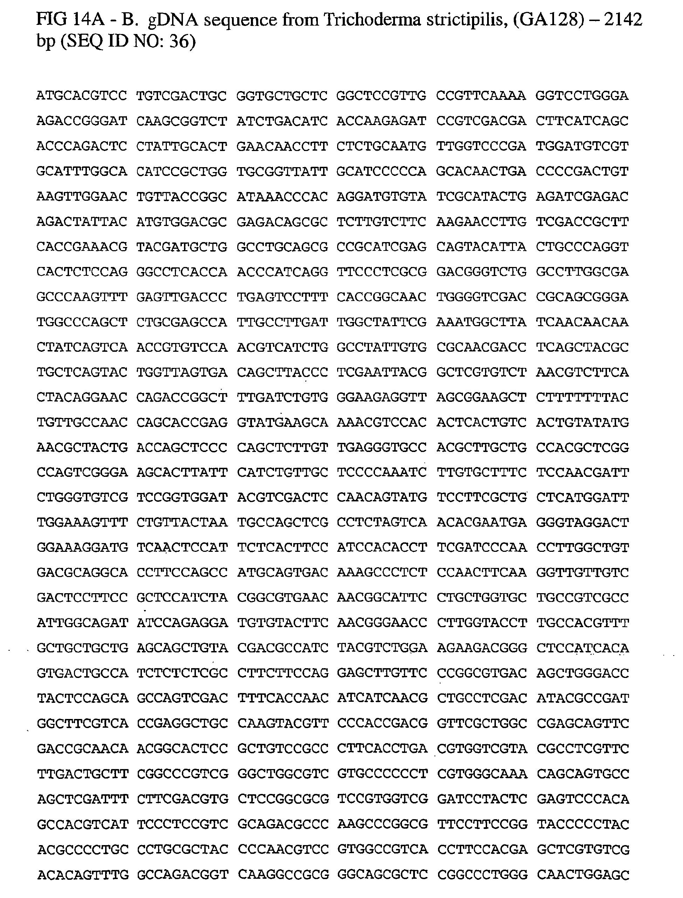

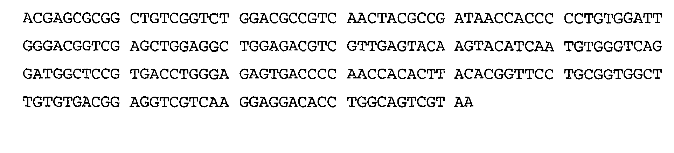

FIG. 14 shows the genomic DNA sequence having 2142 bp (SEQ ID NO: 36) coding for *Trichoderma strictipilis* glucoamylase (GA128) (SEQ ID NO: 37).

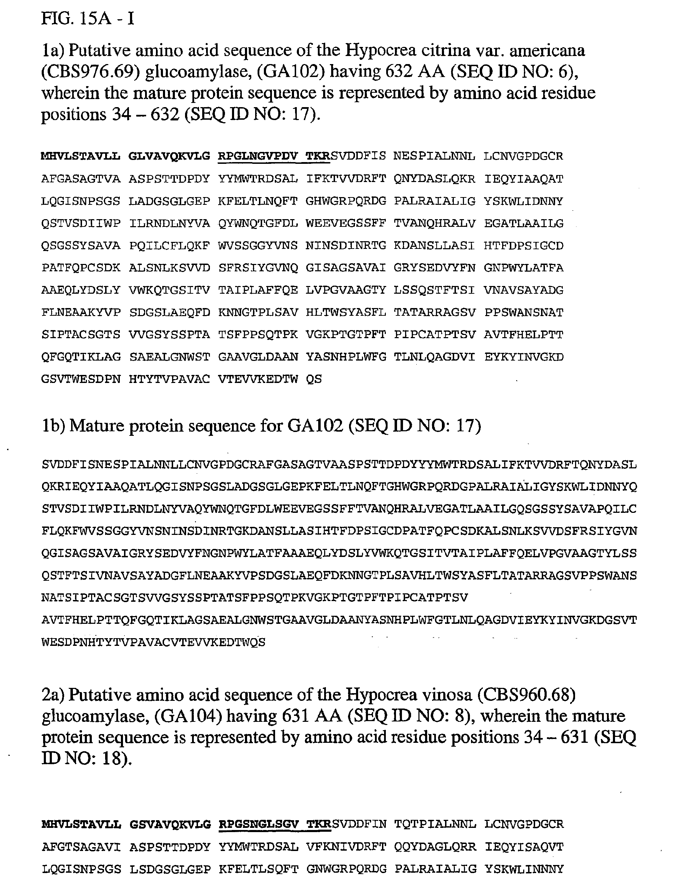

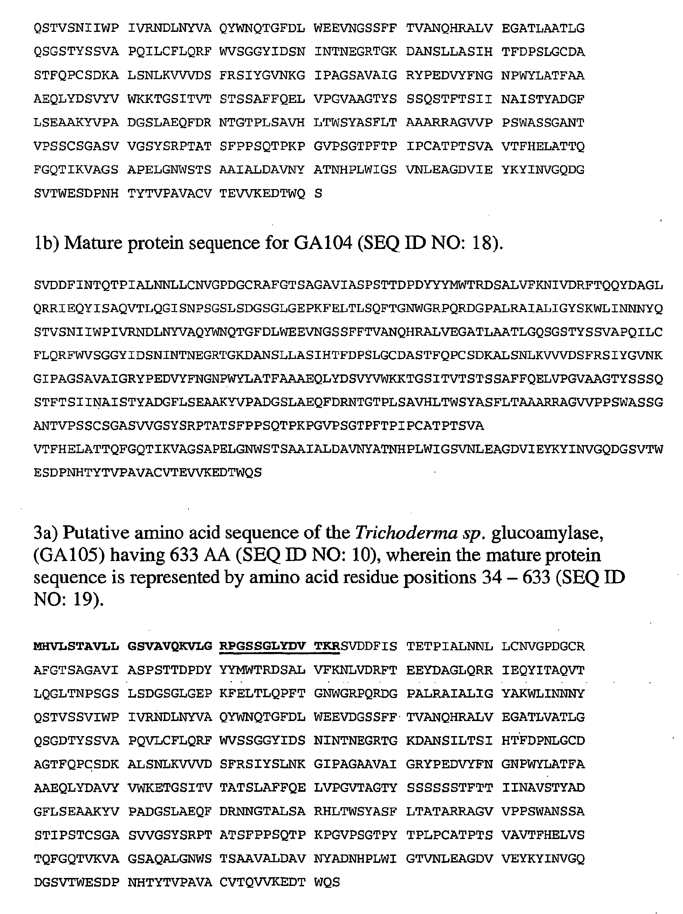

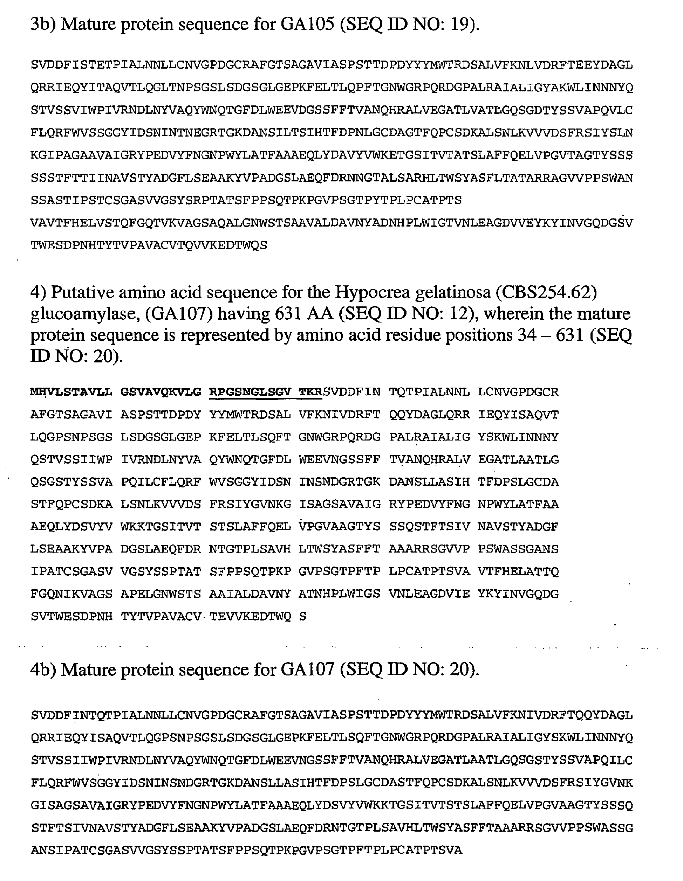

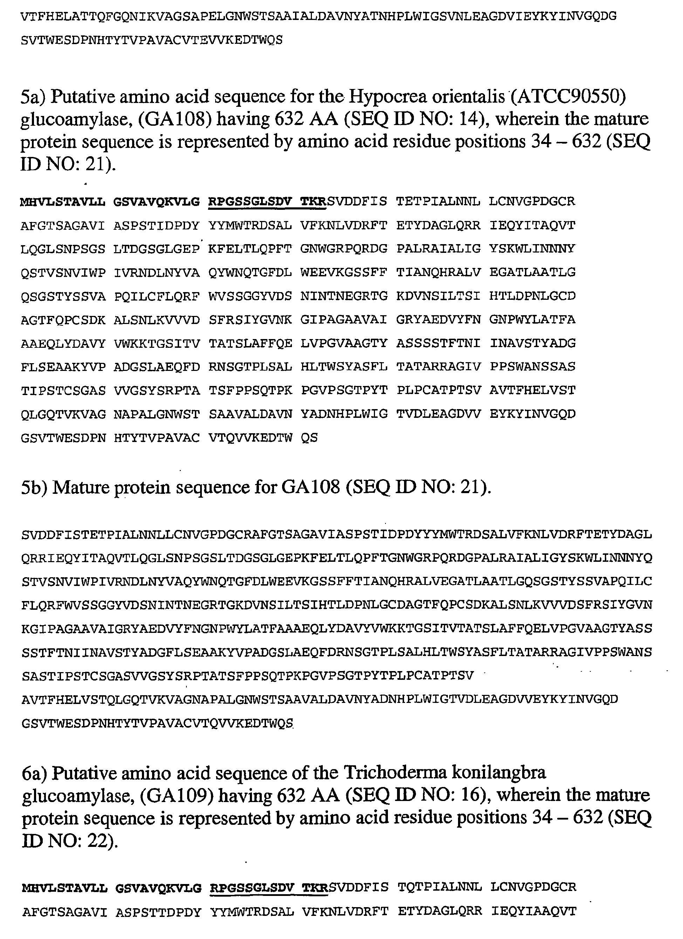

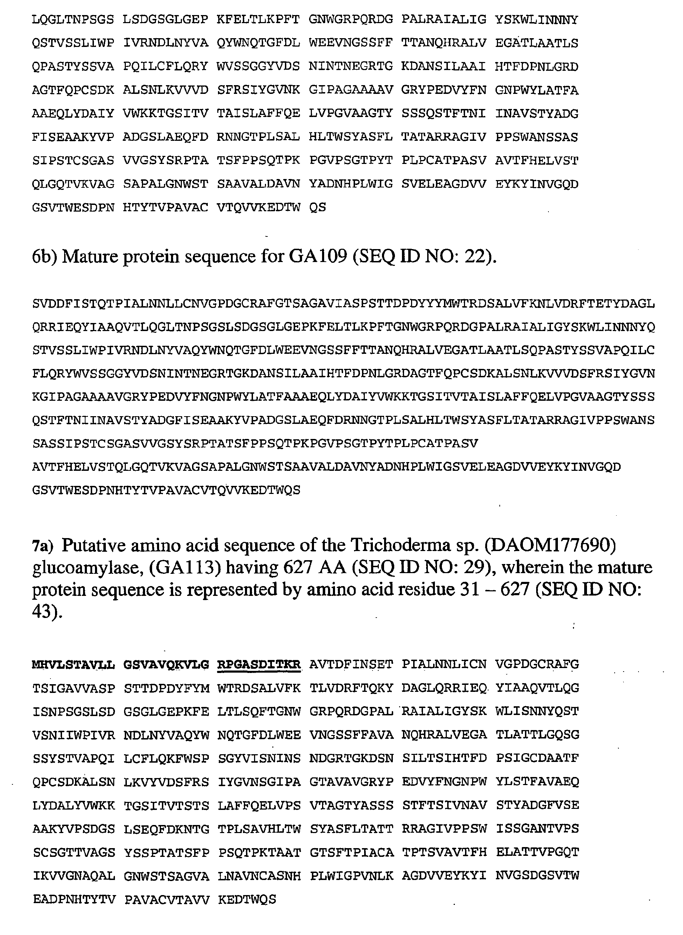

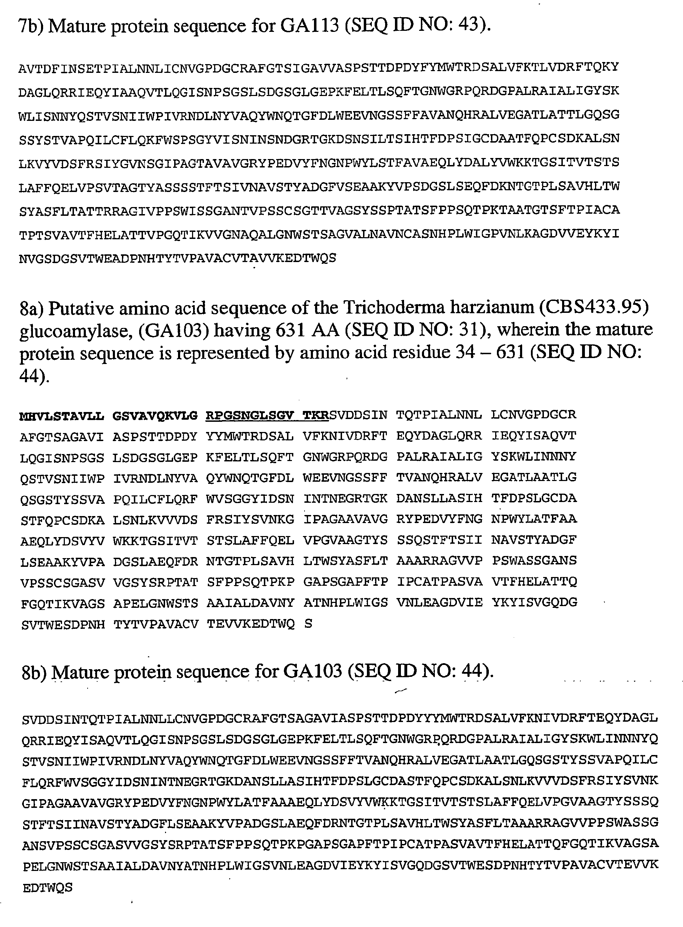

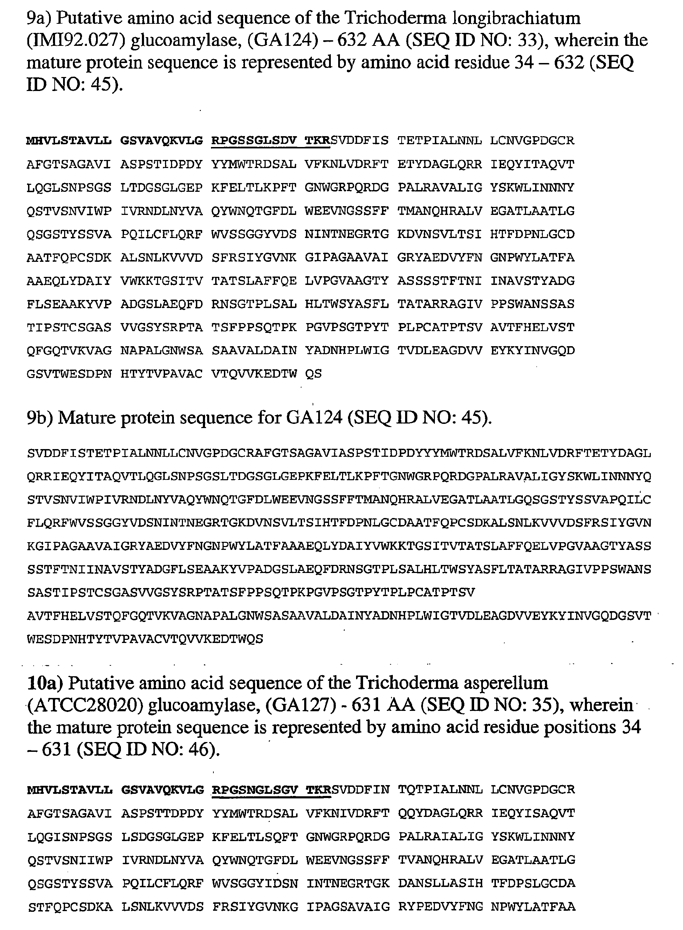

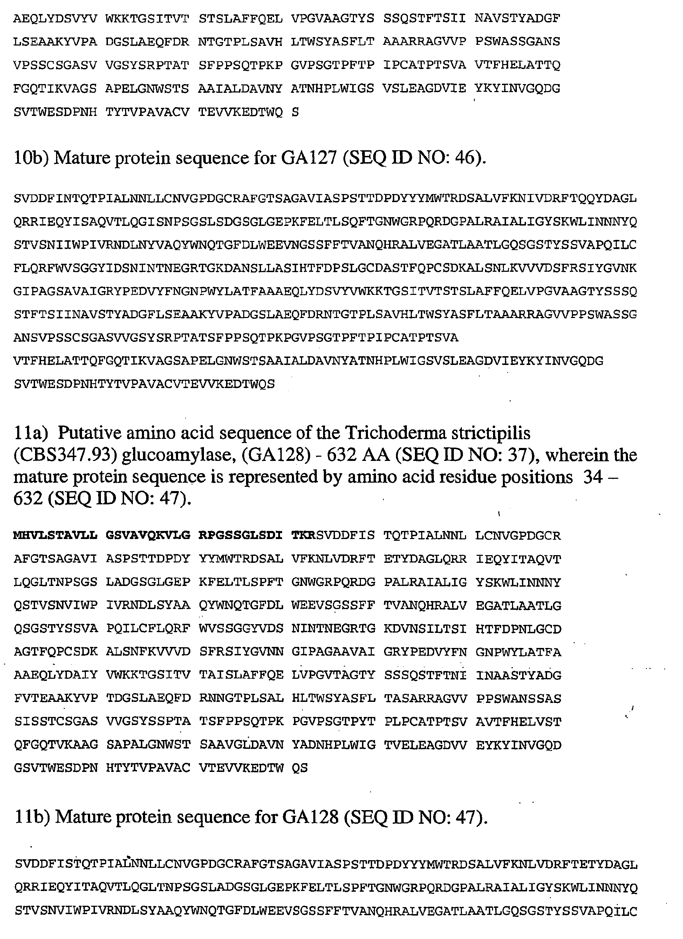

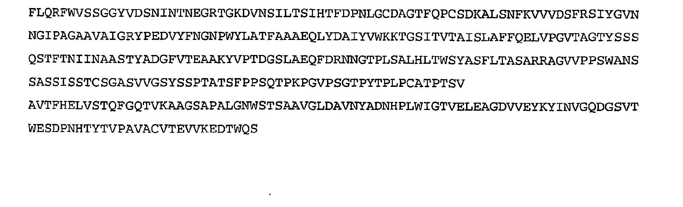

FIG. 15A - I shows the putative amino acid sequences for glucoamylases encoded by the DNA sequences of SEQ ID NOs: 5, 7, 9, 11, 13, 15, 28, 30, 32, 34 and 36, which correspond to the amino acid sequences of SEQ ID NOs: 6, 8, 10, 12, 14, 16, 29, 31, 33, 35 and 37 respectively, wherein the leader peptide is in bold and the prosequence is underlined and in bold for each protein. The mature protein sequence which excludes the leader and prosequence for each protein is also represented as SEQ ID NO: 17 for (1) GA102; SEQ ID NO: 18 for (2) GA104; SEQ ID NO: 19 for (3) GA105; SEQ ID NO: 20 for (4) GA107; SEQ ID NO: 21 for (5) GA108; SEQ ID NO: 22 for (6) GA109; SEQ ID NO: 43 for (7) GA113; SEQ ID NO: 44 for (8) GA103; SEQ ID NO: 45 for (9) GA124; SEQ ID NO: 46 for (10) GA127 and SEQ ID NO: 47 for (11) GA128.

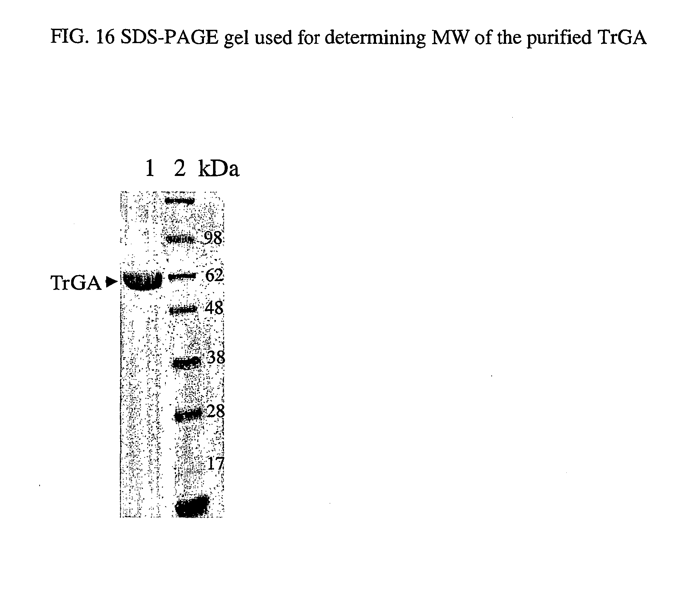

FIG. 16 illustrates the SDS-PAGE gel used for determining MW of the purified TrGA, wherein lane 1 exhibits the TrGA and lane 2 exhibits the molecular weight marker SeeBlue Plus 2 (Invitrogen).

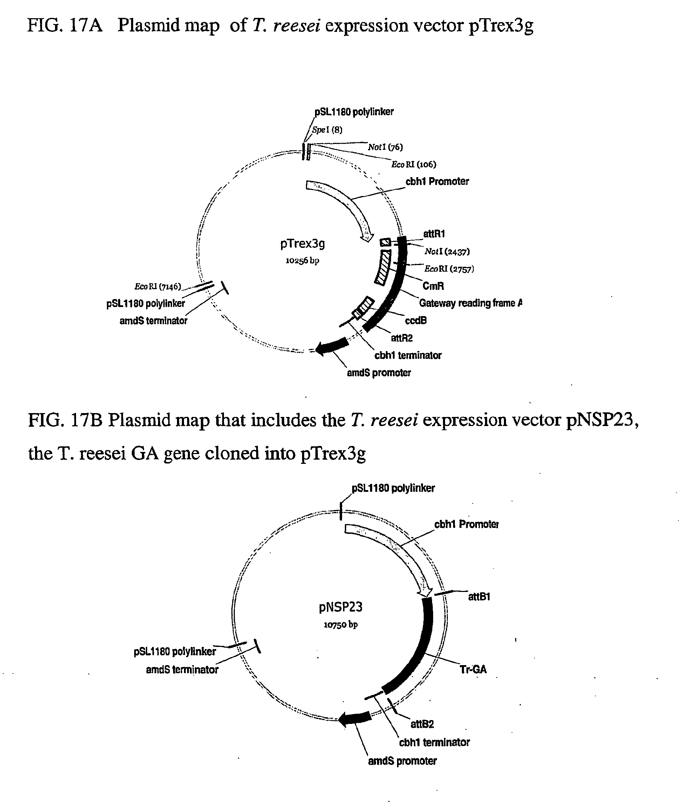

FIG. 17A is a plasmid map of *T. reesei* expression vector, pTrex3g.
FIG 17B is a plasmid map that includes the *T. reesei* expression vector pNSP23, wherein the TrGA gene is cloned into pTrex3g.

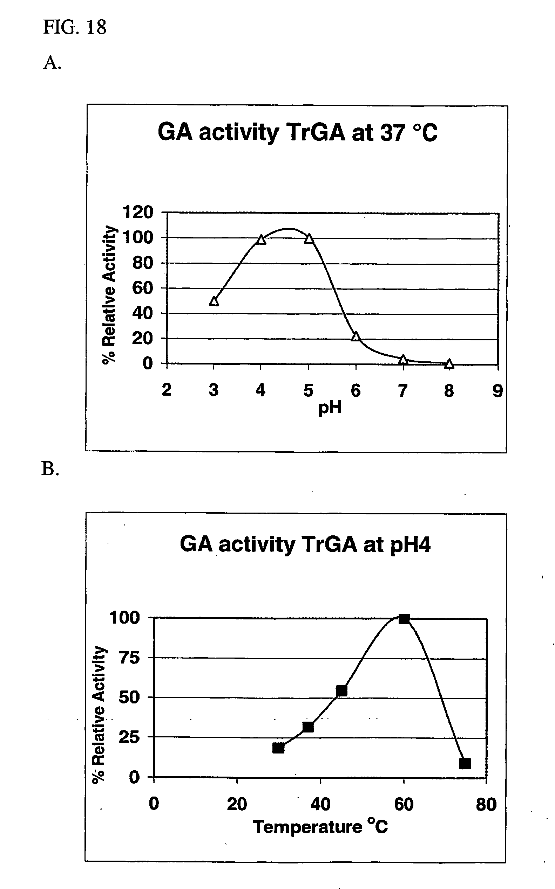

FIG. 18 shows (A) the % relative GA'activity of the TrGA at 37°C from pH 3 - 8 and (B) the % relative GA activity of the TrGA at pH 4.0 from 25°C to 78°C and reference is made to example 4.

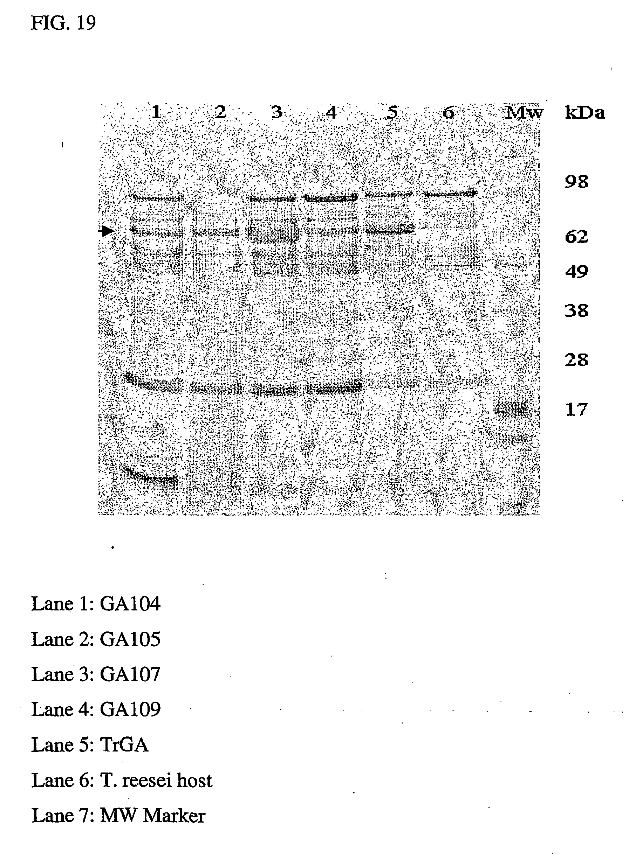

FIG. 19 illustrates the SDS-PAGE gel used for determining secretion of substantially homologous glucoamylases in the Trichoderma host strain (1A52), wherein the band at about 62kDa represents glucoamylase and lane 1 represents GA104, lane 2 represents GA105; lane 3 represents GC107; lane 4 represents GA109; lane 5 represents TrGA; lane 6 represents a Trichoderma reesei control host strain (1A52); and lane 7 represents a standard molecular weight marker.

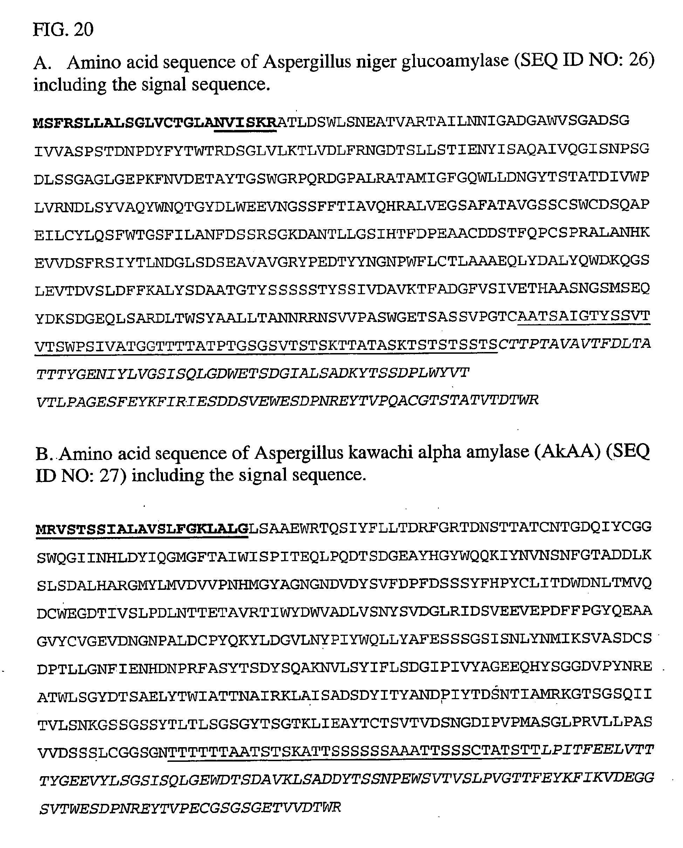

FIG. 20 (A) illustrates the amino acid sequence (SEQ ID NO: 26) for an *Aspergillus niger* glucoamylase which includes the leader sequence. The N- terminal amino acid residue of the mature protein is represented by residue position 25, A (alanine); the linker region is underlined and the starch binding domain is in italics. (B) illustrates the amino acid sequence for an *Aspergillus kawachi* alpha amylase (SEQ ID NO: 27) which includes the leader sequence, wherein the leader sequence is in bold and underlined and is represented by amino acid residues 1 - 21; the linker region is underlined and the starch binding domain is in italics. The mature protein includes the catalytic domain, the linker and the starch binding domain.

### DETAILED DESCRIPTION OF THE INVENTION

In some aspects, the present invention relies on routine techniques and methods used in the field of genetic engineering and molecular biology. The following resources include descriptions of general methodology useful in accordance with the invention: Sambrook et al. Eds., MOLECULE CLONING: A LABORATORY MANUAL (3rd Ed. 2000); Kriegler M. Ed., GENE TRANSFER AND EXPRESSION: A LABORATORY MANUAL (1990); and Ausubel et al. Eds., SHORT PROTOCOLS IN MOLECULAR BIOLOGY (5th Ed. 2002). Although any methods and materials similar or equivalent to those described herein can be used in the practice or testing of the invention, the preferred methods and materials are described below.

Unless defined otherwise herein all technical and scientific terms used herein have the same meaning as commonly understood by one of ordinary skill in the art to which this invention belongs. Singleton et al., et al., DICTIONARY OF MICROBIOLOGY AND MOLECULAR BIOLOGY 2nd Ed, John Wiley and Sons, NY (1994) and Hale and Margham, THE HARPER COLLINS DICTIONARY OF BIOLOGY (1991) Addison Wesley Pub. Co. provides one of skill with dictionaries of many of the terms used in describing this invention.

The invention will now be described in detail by way of reference only using the following definitions and examples.

The singular forms "a", "an" and "the" include the plural references unless the content clearly dictates otherwise. Thus for example, reference to a composition containing "a compound" includes a mixture of two or more compounds. It should be noted that the term "or" is generally employed in the sense including "and/or" unless the content clearly dictates otherwise.

Numeric ranges are inclusive of the numbers of the ranges.

Unless otherwise indicated, nucleic acids are written left to right 5' to 3' orientation; amino acids sequences are written left to right in amino carboxy orientation, respectively.

The headings provided herein are not limitations of the various aspects or embodiments of the invention, which can be had by reference to the specification as a whole.

### Definitions

The term "glucoamylase" refers to the amyloglucosidase class of enzymes (E.C. 3.2.1.3, glucoamylase, 1,4-alpha-D-glucan glucohydrolase). These enzymes release glucosyl residues from the non-reducing ends of amylose and amylopectin molecules.

The phrase "having granular starch hydrolyzing activity" means an enzyme that is capable of hydrolyzing starch in granular form.

The phrase "Trichoderma/Hypocrea family cluster" means a member of the Family Hypocreaceae including several anamorphs as Trichoderma and Gliocladium of the Order Hypocreales, Phylum Ascomycota and reference is made to Chapter 12, Alexopoulos, C.J., et al., in INTRODUCTORY MYCOLOGY 4th Edition, John Wiley & Sons, NY 1996.

The terms "nucleic acid sequence" and "polynucleotide" maybe used interchangeably herein. The term encompasses genomic DNA, intronless DNA, synthetic origins or combinations thereof.

The term "intron" means an intervening DNA sequence that is transcribed but is removed from within the transcript by splicing together the coding sequences of the mature protein.

The term "isolated nucleic acid sequence" means a nucleic acid sequence, which is essentially free of other nucleic acid sequences.

The term "biologically functional fragments of a sequence" (*e.g*. biologically functional fragments of SEQ ID NO: 4) means a polypeptide having glucoamylase activity and one or more amino acid residues deleted from the amino and/or carboxyl terminus of the amino acid sequence.

The term "vector" means a polynucleotide sequence designed to introduce nucleic acids into one or more cell types.

The term "expression vector" means a DNA construct comprising a nucleic acid sequence, which is operably linked to a suitable control sequence capable of effecting expression of the nucleic acid sequence in a suitable host. Suitable control sequences include promoters to effect transcription, operator sequences, sequences encoding suitable ribosome binding sites on the mRNA, enhancers and/or termination sequences.

The term "promoter" means a regulatory sequence involved in binding RNA polymerase to initiate transcription of a gene.

The term "operably linked" refers to juxtaposition wherein the elopements are in an arrangement allowing them to be functionally related. For example, a promoter is operably linked to a coding sequence if it controls the transcription of the sequence.

The term "an isolated polypeptide" means a polypeptide that is essentially free of other non-glucoamylase polypeptides. An isolated polypetide may be at least 20% pure, at least 40% pure, at least 60% pure, at least 70% pure, at least 80% pure, at least 90% pure, at least 95% pure as determined by SDS-PAGE.

The term "signal sequence" means a sequence of amino acids bound to the N-terminal portion of a protein, which facilities the secretion of the mature form of a protein outside the cell. The definition of a signal sequence is a functional one. The mature form of the extracellular protein lacks the signal sequence, which is cleaved off during the secretion process. The terms "signal sequence", signal peptide" and "leader peptide" may be used interchangeability herein. In general the signal sequence refers to the nucleotide sequence and the term leader peptide refers to the amino acid sequence.

The terms "protein" and "polypeptide" are used interchangeably herein. The conventional one-letter or three-letter code for amino acids residues is used herein.

The term "catalytic domain" refers to a structural region of a polypetide, which contains the active site for substrate hydrolysis.

The term "linker" refers to a short amino acid sequence generally having between 3 and 40 amino acids residues that covalently bind an amino acid sequence comprising a starch binding domain with an amino acid sequence comprising a catalytic domain.

The term "starch binding domain" refers to an amino acid sequence that binds preferentially to a starch substrate.

The term "allelic variants" means any of two or more alterative forms of a gene occupying the same chromosomal locus. Allelic variation arises naturally through mutation and may result in polymorphism between populations. An allelic variant of a polypeptide is a polypeptide encoded by an allelic variant of a gene.

The term "host cell" or "host strain" means a suitable host for an expression vector or DNA construct comprising a polypeptide encoding a glucoamylase encompassed by the invention. Suitable host cells are used advantageously in the recombinant production of the glucoamylases encompassed by the invention.

As used herein the term "derived from" used in connection with a polynucleotide or polypeptide means the polypeptide or polynucleotide is native to the microorganism.

The term "heterologous" with reference to a polynucleotide or protein refers to a polynucleotide or protein that does not naturally occur in a host cell.

The term " endogenous" with reference to a polynucleotide or protein refers to a polynucleotide or protein that occurs naturally in a host cell.

The term "expression" means the process by which a polypeptide is produced based on the nucleic acid sequence of a gene.

The term "over expression" means the process of expressing a polypeptide is a host cell wherein a polynucleotide has been introduced the host cell.

The term "introduced" in the context of inserting a nucleic acid sequence into a cell means transfection, transformation or transduction and includes reference to the incorporation of the nucleic acid sequence into a host cell.

The term "granular starch" refers to raw uncooked starch (*e.g*. granular starch that has not been subject to gelatinization).

The term "starch" refers to any material comprised of the complex polysaccharide carbohydrates of plant, comprised of amylose and amylopectin with the formula (C₆H₁₀O₅)ₓ, wherein X can be any number.

The term "gelatinization" means the solubilization of a starch molecule by cooking to form a viscous suspension. The phrase "below the temperature of gelatinization" refers to a temperature less than the temperature which starts gelatinization.

The term "culturing" refers to growing a population of microbial cells under suitable conditions in a liquid or solid medium. In one embodiment, culturing refers to fermentative bioconversion of a starch substrate to an end-product (typically in a vessel or reactor). Fermentation is the enzymatic and anaerobic breakdown of organic substances by microorganisms to produce simpler organic compounds. While fermentation occurs under anaerobic conditions it is not intended that the term be solely limited to strict anaerobic conditions as fermentation also occurs in the presence of oxygen.

The term "end-product" refers to any carbon source derived molecule product which is enzymatically converted from a starch substrate.

The term" enzymatic conversion" refers to the modification of a substrate by enzyme action.

The term "specific activity" means an enzyme unit defined as the number of moles of substrate converted to product by an enzyme preparation per unit time under specific conditions. Specific activity is expressed as units (U)/mg or protein.

The term "monosaccharide" means a monomeric unit of a polymer such as starch wherein the degree of polymerization (DP) is 1 (*e.g*., glucose, mannose, fructose and galactose).

The term "disaccharide" means a compound that comprises two covalently linked monosaccharide units (DP2). The term encompasses, but is not limited to such compounds as sucrose, lactose and maltose.

The term "a DP >3" means polymers with a degree of polymerization greater than 3.

The term "oligosaccharide" means a compound having 2 - 10 monosaccharide units joined in glycosidic linkages.

The term "polysaccharide" means a compound having multiple monosaccharide units joined in a linear or branched chain. In some embodiments the term refers to long chains with hundreds or thousands of monosaccharide units. Typical examples of polysaccharides are starch, cellulose and glycogen.

As used herein the term "dry solids content (DS or ds)" refers to the total solids of a slurry in % on a dry weight basis.

The term "milling" refers to the breakdown of cereal grains to smaller particles. In some embodiments the term is used interchangeably with grinding.

The term "dry milling" refers to the milling of dry whole grain, wherein fractions of the grain such as the germ and bran have not been purposely removed.

As used herein the terms "distillers dried grain (DDG)" and "distillers dried grain with solubles (DDGS)" refer to useful co-products of grain fermentation processes.

The term "DE" or "dextrose equivalent" is an industry standard for measuring the concentration of total reducing sugars, calculated as D-glucose on a dry weight basis. Unhydrolyzed granular starch has a DE that is essentially 0 and D-glucose has a DE of 100.

The term "sugar syrup" refers to an aqueous composition containing soluble carbohydrates. In one embodiment, the sugar syrup is a syrup containing glucose.

### Trichoderma reesei Glucoamylase amino acid sequences

A glucoamylase derived from *Trichoderma reesei* QM6a (ATCC, Accession No. 13631) has been cloned as further described in detail in Example 1. The full length glucoamylase derived from *Trichoderma reesei* is illustrated in Figure 3 and has an amino acid sequence of SEQ ID NO: 3. The mature protein sequence of the *Trichoderma reesei* glucoamylase, (SEQ ID NO: 4) is represented by amino acid residues 34 - 632 of Figure 3.

This invention relates to an enzyme composition comprising a glucoamylase having an amino acid sequence at least 90% identical to SEQ ID NO:4 and an alpha-amylase having at least 90% identity to the mature protein sequence of SEQ ID NO:27

Also described herein is a glucoamylase comprising the sequence shown in SEQ ID NO: 3 or an enzyme with glucoamylase activity being substantially homologous thereto. The glucoamylase of SEQ ID NO: 3 includes the signal sequence of the glucoamylase obtained from *Trichoderma reesei.*

Also described herein is a polypeptide having glucoamylase activity comprising the catalytic domain of the glucoamylase of SEQ ID NO: 4, which is also represented by SEQ ID NO: 40.

### Homology of the Protein Sequence

The homology between two glucoamylases may be determined by the degree of identity between the amino acid sequences of two protein sequences. A polypeptide or polynucleotide having a certain percent of identity with another sequence (i.e. 80%, 90%, and 95%) means that when aligned, that percent of bases or amino acid residues are the same in comparing the two sequences. This alignment and percent homology or identity can be determined by using any suitable software program known in the art. For example suitable programs are described in CURRENT PROTOCOLS IN MOLECULAR BIOLOGY (Ausubel et al., eds 1995, Chapter 19). Preferred programs include GCG Pileup program (Wisconsin Package, Version 8.1 and 10.0), FASTA, BLAST and TFASTA. Another preferred alignment program is ALIGN or ALIGN Plus (Dayhoff (1978) in ATLAS OF PROTEIN SEQUENCE AND STRUCTURE 5: Suppl. 3 (National Biomedical Research Foundation)) Further BLASTP, BLASTN and BLASTX algorithms can be used (Altschul et al., (1990) J. Mol. Biol. 215:403-410). Other useful methods include ClustralW (Thompson et al., (1997) Nucleic Acid Research 25:4.876 -4882) using software provide by DNASTAR (Madison WI). Also reference is made to Needleman et al., (1970) J. Mol. Biol. 48:443, Smith et al., (1981) Adv. Appl. Math. 2: 482, Smith et al., (1997) Meth. Mol. Biol. 70:173-187 and Pearson et al., (1988) Proc. Natl. Acad. Sci. 85:24444.

According to the invention a "substantially homologous" amino acid sequence exhibits glucoamylase activity and at least 90%, at least 93%, at least 95%, at least 97%, at least 98% and at least 99% identity with the sequence illustrated in SEQ ID NO: 4 . Particularly preferred substantially homologous glucoamylase sequences are the mature protein sequences as shown in Figure 15 and which correspond to SEQ ID NOs: 17, 18, 19, 20, 21, 22, 43, 44, 45, 46 and 47. Additionally, preferred substantially homologous glucoamylase sequences are the sequences shown in Figure 15, which correspond to SEQ ID NOs: 6, 8, 10, 12, 14, 16, 29, 31, 33, 35 and 37 and include a leader sequence. Further substantially homologous polypeptides include allelic variations and natural mutants having glucoamylase activity. the invention, the specific activity of the glucoamylases tested under essentially the same conditions will be at least 90%, at least 100%, at least 125%, at least 150%, at least 175% and also at least 200% of the specific activity of the mature protein derived from *Trichoderma reesei* having the sequence illustrated in Figure 3 (SEQ ID NO: 4). In some embodiments, the specific activity may be measured on a soluble starch substrate and in other embodiments the specific activity may be measured on a granular starch substrate.

As described herein, an amino acid sequence having at least 90% sequence identity to the sequence of SEQ ID NO: 3 or SEQ ID NO: 4 may include conservative amino acid substitutions using L-amino acids, wherein one amino acid is replaced by another biologically similar amino acid. Conservative amino acid substitutions are those that preserve the general charge, hydrophobicity/hydrophilicity, and/or steric bulk of the amino acid being substituted. Non-limiting examples of conservative substitutions include those between the following groups: Gly/Ala, Val/Ile/Leu, Lys/Arg, Asn/Gln, Glu/Asp, Ser/Cys/Thr and Phe/Trp/Tyr. Other conservative substitutions can be taken from the table below.

**Table 1 - Conservative Amino Acid Replacements**

| For Amino Acid | Code | Replace with any of |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Om, D-Om |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Om, D-Om |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

The amino acid substitutions may be non-conservative substitutions.

A glucoamylase may be derived from a filamentous fungal strain and particularly substantially homologous sequences will be obtained from strains of the genus *Aspergillus* spp., *Rhizopus* spp., *Humicola* spp., *Fusarium* spp., *Mucor* spp., *Trichoderma* spp., and the like. In a preferred embodiment, substantially homologous sequences having glucoamylase activity will be derived from strains of the *Trichoderma*/*Hypocrea* family cluster. Some of these species include *T. stromaticum, H. citrina* var. *americana, H. citrina, H. lactea, H. hunua, T. fertile, T. tomentosum, H. vinosa, T. harzianum, T. inhamatum, T. oblongisporum, T.* cf. *aureoviride, T. cf. harzianum, T. fasciculatum, H. tawa, T. crassum, T. flavovirens, T. virens, T. longipilis, T. spirale, T. strictipilis, H. pilulifera, T. polysporum, T. croceum, T. minutisporum, T. hamatum, T. asperellum, T. atroviride, T. koningii, T. viride, H. gelatinosa, T. strigosum, T. pubescens, H. novazelandiae, T. saturnisporum, T. longibrachiatum, H. orientalis, T. citrinoviride, T. reesei, T. ghanense, T. .pseudokonimgii, H. andinensis* and *H. aureoviride.* Particularly preferred strains of the genus *Trichoderma* and allied *Hypocrea spp.* include *H. citrina* var. *americana, H. citrina, H. lactea, H. vinosa, T. harzianum, T. atroviride, T. koningii, T. viride, H. gelatinosa, T. saturnisporum, T. longibrachiatum, H. orientalis, T. citrinoviride, T. reesei,* and *T. konilangbra.*

Some strains of the species described above are accessible to the public from culture collections such as American Type Culture Collection (ATCC) P. O. Box 1549, Manassas, VA 20108; Deutsche Sammlung von Mikroorganismen und Zellkulturen GmbH (DSM); Agricultural Research Service Plant Culture Collection, Northern Regional Research Center (NRRL); the Centraalbureau voor Schimmelcultures (CBS), P. O. Box 85167, 3508 AD Utrecht, The Netherlands; Plant Research Institute, Department of Agriculture, Mycology, Ottawa, (DAOM) Canada and International Mycological Institute (IMI), Genetic Resources Collection, Egham, United Kingdom..

Cloned *Trichoderma reesei* and Substantially Homologous DNA sequences Described herein is a cloned DNA sequence coding for a polypeptide exhibiting glucoamylase activity of the invention, said DNA sequence comprising
a) the DNA sequence illustrated in SEQ ID NO: 1;
b) the DNA sequence illustrated in SEQ ID NO: 2;
c) a DNA sequence encoding a glucoamylase having at least 80%, at least 83%, at least 85%, at least 87%, at least 90%, at least 93%, at least 95%, at least 97%, at least 98% and at least 99% identity with the sequence of SEQ ID NO: 3;
d) a DNA sequence encoding a glucoamylase having at least 80%, at least 83 %, at least 85%, at least 87%, at least 90%, at least 93%, at least 95%, at least 97%, at least 98% and at least 99% identity with the sequence of SEQ ID NO: 4;
e) a DNA sequence encoding an enzyme having glucoamylase activity, wherein the enzyme has at least 95%, at least 96%, at least 97%, at least 98% and at least 99% sequence identity to any one of the sequences shown in SEQ ID NOs: 17, 18, 19, 20, 21, 22, 43, 44, 45, 46 and 47;
f) a DNA sequence encoding a biologically functional fragment of a sequence having at least 85%, at least 90%, at least 95%, at least 96%, at least 97% and at least 98% identity to amino acid residue position 1 to 453 of the sequence shown in SEQ ID NO: 4;
g) a DNA sequence encoding an enzyme having glucoamylase activity comprising an amino acid sequence having at least 90%, at least 95%, at least 97% and at least 98% sequence identity to any one of the following sequences
   a. amino acid residue positions 1 to 453 of SEQ ID NO: 17;
   b. amino acid residue positions 1 to 452 of SEQ ID NO: 18;
   c. amino acid residue positions 1 to 454 of SEQ ID NO: 19;
   d. amino acid residue positions 1 to 452 of SEQ ID NO: 20;
   e. amino acid residue positions 1 to 453 of SEQ ID NO: 21;
   f. amino acid residue positions 1 to 453 of SEQ ID NO: 22;
   g. amino acid residue positions 1 to 452 of SEQ ID NO: 43;
   h. amino acid residue positions 1 to 452 of SEQ ID NO: 44;
   i. amino acid residue positions 1 to 453 of SEQ ID NO: 45;
   j. amino acid residue positions 1 to 452 of SEQ ID NO: 46; and
   k. amino acid residue positions 1 to 453 of SEQ ID NO: 47.
h) a DNA which is at least 80%, at least 85%, at least 90%, at least 93%, at least 95%, at least 97% and at least 99% identical to the sequence shown in SEQ ID NO: 1 or SEQ ID NO: 2, wherein said DNA sequence codes for an enzyme having glucoamylase activity; or
i) a DNA sequence, which hybridizes under high stringent conditions to a nucleic acid probe corresponding to the DNA sequence of SEQ ID NO: 2 or a fragment thereof having at least 20, at least 30 at least 40, at least 50 at least 60, at least 70 at least 100, at least 150 consecutive nucleotides.

Also described herein is a cloned DNA sequence encoding an enzyme having glucoamylase activity and at least 95%, at least 96%, at least 97% at least 98% and at least 99% sequence identity to the amino acid sequences of any one of SEQ ID NOs: 6, 8, 10, 12, 14, 16, 29, 31, 33, 35, and 37.

Because of the degeneracy of the genetic code, more than one codon may be used to code for a particular amino acid. Therefore, different DNA sequences may encode a polypeptide having exactly the same amino acid sequence as the polypeptide of, for example SEQ ID NO: 4. Also described are polynucleotides, which encode the same polypeptide. DNA sequences, which encode glucoamylases encompassed by the invention may or may not include introns.

Homology of DNA sequences is determined by the degree of identity between two DNA sequences. Homology may be determined using computer programs as described above for determining protein sequence homology.

A nucleic acid is hybridizable to another nucleic acid when a single stranded form of the nucleic acid can anneal to the other nucleic acid under appropriate conditions of temperature and solution ionic strength. Hybridization and washing conditions are well known in the art for hybridization under low, medium, medium/high, high and very high stringency conditions (See., *e.g*. Sambrook et al., *supra,* particularly chapters 9 and 11). In general, hybridization involves a nucleotide probe and a homologous DNA sequence that form stable double stranded hybrids by extensive base-pairing of complementary polynucleotides (See, Chapter 8, GeneCloning, An Introduction, T.A. Brown, (1995) Chapman and Hall, London).

The filter with the probe and homologous sequence are washed in 2x sodium chloride /sodium citrate (SSC), 0.5%SDS at about 60°C (medium stringency); 65°C (medium/high stringency) 70°C (high stringency) and about 75°C (very high stringency).

### Vectors

Described herein is to a DNA construct comprising a nucleic acid sequence encoding a glucoamylase encompassed by the invention and operably linked to a promoter sequence is assembled to transfer into a host cell. The DNA construct may be introduced into a host cell using a vector. The vector may be any vector which when introduced into a host cell is integrated into the host cell genome and is replicated. Vectors include cloning vectors, expression vectors, shuttle vectors, plasmids, phage particles, cassettes and the like. The vector may be an expression vector that comprises regulatory sequences operably linked to the glucoamylase coding sequence.

Examples of suitable expression and/or integration vectors are provided in Sambrook *et al.,* (1989) *supra,* and Ausubel (1987) *supra,* and van den Hondel et al. (1991) in Bennett and Lasure (Eds.) MORE GENE MANIPULATIONS IN FUNGI, Academic Press pp. 396-428 and U.S. Patent No. 5,874,276. Reference is also made to the Fungal Genetics Stock Center Catalogue of Strains (FGSC, < www.fgsc.net>) for a list of vectors. Particularly useful vectors include vectors obtained from for examples Invitrogen and Promega. Specific vectors suitable for use in fungal host cells include vectors such as pFB6, pBR322, pUC18, pUC100, pDON^{™}201, pDONR^{™}221, pENTR^{™}, pGEM^{®}3Z and pGEM^{®}4Z.

The promoter, which shows transcriptional activity in a fungal host cell may be derived from genes encoding proteins either homologous or heterologous to the host cell. The promoter may be a mutant, truncated and hybrid promoter. Preferably, the promoter is useful in a *Trichoderma* or *Aspergillus* host. Exemplary promoters include the T. reesei promoters *cbh*1, *cbh*2, *egl*1, *egl*2, *eg*5, *xln*1 and *xln*2. Other examples of useful promoters include promoters from *A. awamori* and *A. niger* glucoamylase genes (glaA) (*See,* Nunberg et al., (1984) Mol. Cell Biol. 4:2306-2315 and Boel et al., (1984) EMBO J. 3:1581-1585), *Aspergillus nidulans* acetamidase genes and *Rhizomucor miehei* lipase genes.

The promoter may be native to the host cell. For example, when *T. reesei* is the host, the promoter is a native *T. reesei* promoter.

Alternatively, the promoter may be heterologous to the fungal host cell.

The promoter may be *T. reesei cbh1*, which is an inducible promoter and has been deposited in GenBank under Accession No. D86235.

An "inducible promoter" is a promoter that is active under environmental or developmental regulation. The DNA construct may include nucleic acids coding for a signal sequence that is an amino acid sequence linked to the amino terminus of the polypeptide which directs the encoded polypeptide into the cell's secretory pathway. The 5' end of the coding sequence of the nucleic acid sequence may naturally include a signal peptide coding region which is naturally linked in translation reading frame with the segment of the glucoamylase coding sequence which encodes the secreted glucoamylase or the 5' end of the coding sequence of the nucleic acid sequence may include a signal peptide which is foreign to the coding sequence. The DNA construct may include a signal sequence that is naturally associated with the glucoamylase gene to be expressed. Effective signal sequences may include the signal sequences obtained from glucoamylases of other filamentous fungal cells, such as from *Humicola, Aspergillus, and Rhizopus.*

The nucleic acid of the DNA construct codes for a signal sequence having at least 95%, at least 96%, at least 97%, at least 98% and at least 99% sequence identity to the signal sequence depicted in Figure 3.

A DNA construct or vector comprising a signal sequence and a promoter sequence to be introduced into a fungal host cell may be derived from the same source. For example, the signal sequence may be the *cbh*1 signal sequence which is operably linked to a *cbh*1 promoter. Alternatively the native glucoamylase signal sequence of a Trichoderma/Hypocrea family cluster member may be used.

The expression vector may also include a termination sequence. Any terminator sequence functional in the host cell may be used For example, the termination sequence and the promoter sequence may be derived from the same source. The termination sequence may be homologous to the host cell. A particularly suitable terminator sequence is *cbh*1 derived from a *Trichoderma* strain and particularly *T. reesei.* Other useful fungal terminators include the terminator from *A. niger* or *A. awamori* glucoamylase genes (Nunberg *et al.* (1984) *supra,* and Boel *et al*., (1984) *supra*), *Aspergillus nidulans* anthranilate synthase genes, *Aspergillus oryzae* TAKA amylase genes, or *A. nidulans trp*C (Punt et al., (1987) Gene 56:117 - 124).

An expression vector may include a selectable marker. Examples of preferred selectable markers include ones which confer antimicrobial resistance (*e.g*., hygromycin and phleomycin). Nutritional selective markers also find use including those markers known in the art as *amd*S*, arg*B and *pyr4.* Markers useful in vector systems for transformation of *Trichoderma* are known in the art (See, *e.g*., Finkelstein, chapter 6 in BIOTECHNOLOGY OF FILAMENTOUS FUNGI, Finkelstein et al. Eds. Butterworth-Heinemann, Boston, MA (1992), Chap. 6.; and Kinghorn et al. (1992) APPLIED MOLECULAR GENETICS OF FILAMENTOUS FUNGI, Blackie Academic and Professional, Chapman and Hall, London). A selective marker is the *amd*S gene, which encodes the enzyme acetamidase, allowing transformed cells to grow on acetamide as a nitrogen source. The use of *A. nidulans amd*S gene as a selective marker is described in Kelley et al., (1985) EMBO J. 4:475 - 479 and Penttilä et al., (1987) Gene 61:155-164.

Methods used to ligate the DNA construct comprising a nucleic acid sequence encoding a glucoamylase, a promoter, a terminator and other sequences and to insert them into a suitable vector are well known in the art. Linking is generally accomplished by ligation at convenient restriction sites. If such sites do not exist, synthetic oligonucleotide linkers are used in accordance with conventional practice. *(See,* Sambrook (1989) *supra,* and Bennett and Lasure, MORE GENE MANIPULATIONS IN FUNGI, Academic Press, San Diego (1991) pp 70 - 76.). Additionally, vectors can be constructed using known recombination techniques (*e.g*., invitrogen Life Technologies, Gateway Technology).

### Host Cells

Host cells comprising a nucleic acid sequence encoding an enzyme of the invention, which are used in the production of the compositions of the invention, are described herein. Preferred host cells are filamentous fungal cells, and the term host cell includes both the cells, progeny of the cells and protoplasts created from the cells of a filamentous fungal strains.

The term "filamentous fungi" refers to all filamentous forms of the subdivision Eumycotina (*See,* Alexopoulos, C. J. (1962), INTRODUCTORY MYCOLOGY, Wiley, New York). These fungi are characterized by a vegetative mycelium with a cell wall composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism is obligatory aerobic. The filamentous fungal parent cell may be a cell of a species of, but not limited to, *Trichoderma, (e.g., Trichoderma reesei,* the asexual morph of *Hypocrea jecorina, T, longibrachiatum, Trichoderma viride, Trichoderma koningii, Trichoderma harzianum*)*, Penicillium sp., Humicola sp. (e.g., H. insolens, H. lanuginosa* and *H. grisea*); *Chrysosporium sp.* (*e.g., C. lucknowense*), *Gliocladium sp., Aspergillus sp. (e.g., A. oryzae, A. niger, A. nidulans,* and *A. awamori), Fusarium sp.,(e.g. F. graminum* and *F. venenatum), Neurospora sp., Hypocrea sp., Mucor, and Emericella sp.* (See also, Innis et al., (1985) Sci. 228:21 -26). The term *"Trichoderma"* or *"Trichoderma sp."* refer to any fungal genus previously or currently classified as *Trichoderma.* The host cell may be a genetically engineered host cell wherein native genes have been inactivated, for example by deletion. Where it is desired to obtain a fungal host cell having one or more inactivated genes known methods may be used (*e.g*. methods disclosed in U.S. Patent No. 5,246,853, U.S. Patent No. 5,475,101 and WO92/06209). Gene inactivation may be accomplished by complete or partial deletion, by insertional inactivation or by any other means which renders a gene nonfunctional for its intended purpose (such that the gene is prevented from expression of a functional protein). Any gene from a *Trichoderma sp.* or other filamentous fungal host, which has been cloned can be deleted. If the host cell is a *Trichoderma* cell and particularly a *T. reesei* host cells the *cbh*1*, cbh*2*, egl*1 and *egl*2 genes may be inactivated and preferably deleted. Particualrly preferred *Trichoderma reesei* host cells having quad-deleted proteins are set forth and described in USP 5,847,276 and WO 05/001036.

### Transformation of Host Cells

Introduction of a DNA construct or vector into a host cell includes techniques such as transformation; electroporation; nuclear microinjection; transduction; transfection, (*e.g*., lipofection mediated and DEAE-Dextrin mediated transfection); incubation with calcium phosphate DNA precipitate; high velocity bombardment with DNA-coated microprojectiles; and protoplast fusion. General transformation techniques are known in the art (See, *e.g*., Ausubel et al., (1987), *supra,* chapter 9; and Sambrook (1989) *supra,* and Campbell et al., (1989) Curr. Genet. 16:53-56). The expression of heterologous protein in *Trichoderma* is described in USP 6,022,725; USP 6,268,328; Harkki et al. (1991); Enzyme Microb. Technol. 13:227-233; Harkki et al., (1989) Bio Technol. 7:596-603; EP 244,234; EP 215,594; and Nevalainen et al., "The Molecular Biology of Trichoderma and its Application to the Expression of Both Homologous and Heterologous Genes", in MOLECULAR INDUSTRIAL MYCOLOGY, Eds. Leong and Berka, Marcel Dekker Inc., NY (1992) pp. 129 - 148). Reference is also made to Cao et al., (2000) Sci. 9:991 - 1001 and EP 238 023 for transformation of *Aspergillus* strains and WO96/00787 for transformation of *Fusarium* strains.

Preferably, genetically stable transformants are constructed with vector systems whereby the nucleic acid encoding the glucoamylase is stably integrated into a host strain chromosome. Transformants are then purified by known techniques. In one nonlimiting example, stable transformants including an *amd*S marker are distinguished from unstable transformants by their faster growth rate and the formation of circular colonies with a smooth, rather than ragged outline on solid culture medium containing acetamide. Additionally, in some cases a further test of stability is conducted by growing the transformants on solid non-selective medium (*i.e.,* NH₄(SO₄)₂ (5 mg/mL) as a nitrogen source), harvesting spores from this culture medium and determining the percentage of these spores which subsequently germinate and grow on selective medium containing 10mM acetamide as a sole nitrogen source. Alternatively, other methods known in the art may be used to select transformants.

The preparation of *Trichoderma sp.* for transformation may involve the preparation of protoplasts from fungal mycelia *(See,* Campbell et al., (1989) Curr. Genet. 16:53-56). Also agrobacterium tumefaciens-mediated transformation of filamentous fungi is known *(See,* de Groot et al., (1998) Nat. Biotechnol. 16:839 - 842).

The mycelia may be obtained from germinated vegetative spores. The mycelia are treated with an enzyme that digests the cell wall resulting in protoplasts. The protoplasts are then protected by the presence of an osmotic stabilizer in the suspending medium. These stabilizers include sorbitol, mannitol, potassium chloride, magnesium sulfate and the like. Usually the concentration of these stabilizers varies between 0.8 M and 1.2 M. It is preferable to use about a 1.2 M solution of sorbitol in the suspension medium. Uptake of DNA into the host *Trichoderma sp.* strain is dependent upon the calcium ion concentration. Generally, between about 10 mM CaCl₂ and 50 mM CaCl₂, is used in an uptake solution. Reference is also made to USP 6,022,725 and USP 6,268,328 for transformation procedures used with filamentous fungal hosts.

Desribed herein are methods of recombinantly producing the glucoamylase comprising expressing a polynucleotide encoding a glucoamylase of the invention in a filamentous fungal host cell and cultivating the host cell under conditions suitable for production of the glucoamylase and optionally recovering the glucoamylase.

The fungal cells may be cultured under suitable conditions in shake flask cultivation, small scale or large scale fermentations (including continuous, batch and fed batch fermentations) in laboratory or industrial fermentors, with suitable medium containing physiological salts and nutrients (See, *e.g*., Pourquie, J. et al., BIOCHEMISTRY AND GENETICS OF CELLULOSE DEGRADATION, eds. Aubert, J. P. et al., Academic Press, pp. 71-86, 1988 and Ilmen, M. et al., (1997) Appl. Environ Microbiol. 63:1298-1306). Common commercially prepared media (*e.g*., Yeast Malt Extract (YM) broth, Luria Bertani (LB) broth and Sabouraud Dextrose (SD) broth) find use in the present invention. Preferred culture conditions for a given filamentous fungus are known in the art and may be found in the scientific literature and/or from the source of the fungi such as the American Type Culture Collection and Fungal Genetics Stock Center. In cases where a glucoamylase coding sequence is under the control of an inducible promoter, the inducing agent (*e.g*., a sugar, metal salt or antimicrobial), is added to the medium at a concentration effective to induce glucoamylase expression.

In order to evaluate the expression of a glucoamylase by a cell line that has been transformed with a polynucleotide encoding an enzyme encompassed by the invention, assays may be carried out at the protein level, the RNA level and/or by use of functional bioassays particular to glucoamylase activity and/or production. Some of these assays include Northern blotting, dot blotting (DNA or RNA analysis), RT-PCR (reverse transcriptase polymerase chain reaction), or *in situ* hybridization, using an appropriately labeled probe (based on the nucleic acid coding sequence) and conventional Southern blotting and autoradiography.

In addition, the production and/or expression of a glucoamylase may be measured in a sample directly, for example, by assays directly measuring reducing sugars such as glucose in the culture medium and by assays for measuring glucoamylase activity, expression and/or production. In particular glucoamylase activity may be assayed by the 3,5-dinitrosalicylic acid (DNS) method *(See,* Goto et al., (1994) Biosci. Biotechnol. Biochem. 58:49 - 54). Protein expression may be evaluated by immunological methods, such as immunohistochemical staining of cells, tissue sections or immunoassay of tissue culture medium, (*e.g*., by Western blot or ELISA). Such immunoassays can be used to qualitatively and quantitatively evaluate expression of a glucoamylase. The details of such methods are known to those of skill in the art and many reagents for practicing such methods are commercially available.

The enzymes contained in the composition of the present invention may be recovered or purified from culture media by a variety of procedures known in the art including centrifugation, filtration, extraction, precipitation and the like.

### Uses and Compositions

The present invention is also directed to compositions comprising glucoamylases and methods of using the composition in industrial and commercial application as described in the claims Nonlimiting examples, which include the use of glucoamylases encompassed by the invention in industrial and commercial applications are briefly described below.

The enzyme compositions may be used in starch hydrolyzing and saccharifying compositions, cleaning and detergent compositions (*e.g*., laundry detergents, dish washing detergents, and hard surface cleaning compositions), and in animal feed compositions. Further the enzyme compositions may be used in baking applications, such as bread and cake production, brewing, healthcare, textile, environmental waste conversion processes, biopulp processing, and biomass conversion applications.

In particular, the enzyme compositions may may be used for starch conversion processes, and particularly in the production of dextrose for fructose syrups, specialty sugars and in alcohol and other end-product (e.g. organic acid, ascorbic acid, and amino acids) production from fermentation of starch containing substrates (G.M.A van Beynum et al., Eds, (1985) STARCH CONVERSION TECHNOLOGY, Marcel Dekker Inc. NY). Dextrins produced using enzyme compositions of the invention may result in glucose yields of at least 80%, at least 85%, at least 90% and at least 95%. Production of alcohol from the fermentation of starch substrates using enzyme compositions of the invention may include the production of fuel alcohol or portable alcohol.

The glucoamylases of the invention may find use in the hydrolysis of starch from various plant-based substrates, which are used for alcohol production. The plant-based substrates may include corn, wheat, barley, rye, milo, rice, sugar cane and combinations thereof. The plant-based substrate may be fractionated plant material, for example a cereal grain such as corn, which is fractionated into components such as fiber, germ, protein and starch (endosperm) (USP 6,254,914 and USP 6,899,910). Methods of alcohol fermentations are described in THE ALCOHOL TEXTBOOK, A REFERENCE FOR THE BEVERAGE, FUSEL AND INDUSTRIAL ALCOHOL INDUSTRIES, 3rd Ed., Eds K.A. Jacques et al., 1999, Nottingham University Press, UK. The alcohol may be ethanol. In particular, alcohol fermentation production processes are characterized as wet milling or dry milling processes. The compositions may be used in a wet milling fermentation process or a dry milling process.

Dry grain milling involves a number of basic steps, which generally include: grinding, cooking, liquefaction, saccharification, fermentation and separation of liquid and solids to produce alcohol and other co-products. Plant material and particularly whole cereal grains, such as corn, wheat or rye are ground. In some cases the grain may be first fractionated into component parts. The ground plant material may be milled to obtain a coarse or fine particle. The ground plant material is mixed with liquid in a slurry tank. The slurry is subjected to high temperatures in a jet cooker along with liquefying enzymes (*e.g*. alpha amylases) to solubles and hydrolyze the starch in the cereal to dextrins. The mixture is cooled down and further treated with saccharifying enzymes, such as glucoamylases encompassed by the instant invention, to produce glucose. The mash containing glucose is then fermented for approximately 24 to 120 hours in the presence of fermentation microorganisms, such as ethanol producing microorganism and particularly yeast (Saccharomyces spp). The solids in the mash are separated from the liquid phase and alcohol such as ethanol and useful co-products such as distillers' grains are obtained.

The saccharification step and fermentation step may be combined and the process is referred to as simultaneous saccharification and fermentation or simultaneous saccharification, yeast propagation and fermentation.

In some embodiments, the cooking step or exposure of the starch containing substrate to temperatures above the gelatinization temperate of the starch in the substrate may be eliminated. These fermentation processes may include milling of a cereal grain or fractionated grain and combining the ground cereal grain with liquid to form a slurry which is then mixed in a single vessel with a composition according to the invention and optionally other enzymes such as but not limited to other alpha amylases, other glucoamylases and enzymes having granular starch hydrolyzing activity and yeast to produce ethanol and other co-products (USP 4,514,496, WO 04/081193 and WO 04/080923).

In some embodiments, the invention pertains to a method of saccharifying a liquid starch solution, which comprises an enzymatic saccharification step using a composition of the invention.

An enzyme composition encompassed by the invention may be obtained in culture media or recovered and purified from the culture medium and may be optionally used in combination with any one or combination of the following enzymes - alpha amylases, proteases, pullulanases, isoamylases, cellulases, hemicellulases, xylanases, cyclodextrin glycotransferases, lipases, phytases, laccases, oxidases, esterases, cutinases, xylanases, granular starch hydrolyzing enzyme and other glucoamylases.

Compositions of the invention comprise glucoamylases combined with alpha amylases, such as fungal alpha amylases *(e.g. Aspergillus* sp.) or bacterial alpha amylases *(e.g. Bacillus* sp. such as *B. stearothermophilus, B. amyloliquefaciens* and *B. licheniformis)* and variants thereof. As described in claim 1 the alpha amylase will be an alpha amylase having at least 90%, 93%, 95%, 96%, 97%, 98% and 99% sequence identity to the mature protein sequence of SEQ ID NO: 27. Commercially available alpha amylases contemplated for use in the compositions of the invention are known and include GZYME G997, SPEZYME FRED, SPEZYME EHTYL (Genencor International Inc.) and TERMAMYL 120-L and SUPRA (Novozymes, Biotech.).

The compositions of the invention may be combined with other glucoamylase, for example one or more glucoamylases derived from strains of *Aspergillus* or variants thereof, such as *A. oryzae, A. niger* (*e.g.*, the mature protein sequence of Figure 20(A),*A kawachi,* and *A. awamori;* glucoamylases derived from strains of *Humicola* or variants thereof, particualrly *H. grisea,* such as the glucoamylase having at least 90%, 93%, 95%, 96%, 97%, 98% and 99% sequence identity to SEQ ID NO: 3 disclosed in WO 05/052148; glucoamylases derived from strains of *Talaromyces* or variants thereof, particularly *T. emersonii;* and glucoamylases derived from strains of *Athelia* and particularly *A. rolfsii.*

### MATERIAL AND METHODS

In the disclosure and experimental section which follows, the following abbreviations apply:

TrGA (a *Trichoderma reesei* glucoamylase composition, the mature protein having the amino acid sequence of SEQ ID NO: 4); AkAA (an *Aspergillus kawachi* alpha amylase composition having the mature protein of sequence SEQ ID NO: 27); AnGA (DISTILLASE comprising an Aspergillus niger GA (Genencor International Inc.,)); GA (glucoamylase); GAU (glucoamylase unit); AAU (alpha amylase unit); wt% (weight percent); °C (degrees Centigrade); rpm (revolutions per minute); H₂O (water); dH₂O (deionized water); dIH₂O (deionized water, Milli-Q filtration); aa or AA (amino acid); bp (base pair); kb (kilobase pair); kD or kDa (kilodaltons); g or gm (grams); µg (micrograms); mg (milligrams); µL (microliters); ml and mL (milliliters); mm (millimeters); µm (micrometer); M (molar); mM (millimolar); µM (micromolar); U (units); V (volts); MW (molecular weight); sec (seconds); min(s) (minute/minutes); hr(s) (hour/hours); DO (dissolved oxygen); and EtOH (ethanol).

The following assays and methods are used in the examples provided below:
1) GA Assay - Glucoamylase assay: Glucoamylase activity was measure using a well-known assay which is based on the ability of glucoamylase to catalyze the hydrolysis of p-nitrophenyl-alpha-D-glucopyranoside (pNPG) to glucose and p-nitrophenol. At an alkaline pH, the nitrophenol forms a yellow color that is proportional to glucoamylase activity and is monitored at 400nm and compared against an enzyme standard measured as a GAU (Elder, M. T. and Montgomery R. S., Glucoamylase activity in industrial enzyme preparations using colorimetric enzymatic method, Journal of AOAC International, vol. 78(2), 1995).
One GAU is defined as the amount of enzyme that will produce 1 gm of reducing sugar calculated as glucose per hour from a soluble starch substrate (4% ds) at pH 4.2 and 60°C.
2) Primers and PCR protocol for amplification of genes from Trichoderma/Hypocrea strains :

| Trichoderma/Hypocrea GA - gene | Primer | Gene Specific Sequence | SEQ ID NO: |
|---|---|---|---|
| *Trichoderma reesei* | NSP231F | ATGCCCGCCTTCGCCATGGACC | 23 |
| | NSP232R | TTACGACTGCCAGGTGTCCTCC | 24 |
| | NSP233F | ATGCACGTCCTGTCGACTGCGG | 25 |

| Component | µl |
|---|---|
| Forward primer (10µM) | 1 |
| Reverse primer (10µM) | 1 |
| Template genomic DNA | 5 |
| dNTP (10mM) | 1 |
| HiFi Buffer | 5 |
| MgSO₄(50mM) | 2 |
| DNA polymerase- Platinum Taq Polymerase High Fidelity (Invitrogen, cat. No. 11304-029 | 0.5 |
| Milli-Q water, sterile | 34.5 |

With respect to the PCR program, initial denaturation was 2 min, at 94°C for 1 cycle; denaturation 30 sec, at 94°C, annealing for 30 sec, at 55°C and extension for 2 min at 68°C for 30 cycles and a final extension step of 7 min at 68°C.
3) Ethanol and carbohydrate determinations
Ethanol and carbohydrate composition of the samples were determined using the HPLC method as described herein:
a) a 1.5 mL Eppendorf centrifuge tube was filled with fermentor beer and cooled on ice for 10 min;
b) the sample tube was centrifuged for 1 min in Eppendorf table top centrifuge;
c) a 0.5 mL sample of the supernatant was transferred to a test tube containing 0.05 mL of Kill solution (1.1N H₂SO₄) and allowed to stand for 5 min;
d) 5.0 mL of water is added to the test tube sample and then filtered into a HPLC vial through 0.45 µm Nylon Syringe Filter; and
e) run on HPLC.

### HPLC conditions:

a) Ethanol System: Column: Phenomenex Rezex Organic Acid Column (RHM-Monosaccharide) #OOH 0132-KO (Equivalent to Bio-Rad 87H); Column Temperature: 60°C; Mobile Phase: 0.01 N H₂SO_{4;} Flow Rate: 0.6 mL/min; Detector: RI; and Injection Volume: 20 µL.
b) Carbohydrate System: Column: Phenomenex Rezex Carbohydrate (RCM-Monosaccharide) #00H-0130-KO (Equivalent to Bio-Rad 87H); Column Temperature: 70°C ; Mobile Phase: Nanopure DI H₂O Flow Rate: 0.8 mL/min; Detector: RI; Injection Volume: 10 µL (3% DS material)

The column separates based on the molecular weight of the saccharides, which are designated as DP-1 (monosaccharides); DP-2 (disaccharides); DP-3 (trisaccharides) and DP > 3 (oligosaccharide sugars having a degree of polymerization greater than 3).
4) Residual starch iodine test: A sample of the beer (fermentation broth) was centrifuged in 2 ml plastic centrifuge tubes. The supernatant was decanted and the tube containing the pellet was placed in an ice bath. Several drops of 0.025N iodine solution (0.1N iodine from VWR Cat. No. VW3207-1 diluted 4X) was added to the pellet and mixed. A positive (+) starch shows a range of color from blue to purple and the intensity of color is directly proportional to the concentration of starch. A negative result (-) remains yellowish.
5) Determination of total starch content: The enzyme-enzyme starch liquefaction and saccharification process (dual enzyme method) was used to determine the total starch content. In a typical analysis, 2 g of the dry sample was taken in a 100 ml Kohlraucsh flask and 45 ml of MOPS buffer, pH 7.0 was added. The slurry was well stirred for 30 min. SPEZYME FRED (1:50 diluted in water), 1.0 ml was added and heated to boiling for 3-5 min. The flask was placed in an autoclave maintained at 121°C for 15 min. After autoclaving the flask was placed in a water bath at 95°C and 1 ml of 1:50 dilutes SPEZYME FRED was added and incubated for 45 min. The pH was adjusted to pH 4.2 and the temperature was reduced to 60°C. This was followed by addition of 20 ml acetate buffer, pH 4.2. Saccharification was carried out by adding 1.0 ml of 1:100 diluted OPTIDEX L-400 (Glucoamylase from Genencor International Inc.) and the incubation was continued for 18 hr at 60°C. The enzyme reaction was terminated by heating at 95°C for 10 min. The total sugar composition was determined by HPLC analysis using glucose as a standard. The soluble starch hydrolysate from water extraction of a sample at room temperature without enzymatic treatment was subtracted from the total sugar.
6) Total protein analysis: The total nitrogen (N) in the sample preparations was determined using the Kjeldhal method (American Assoc. Cereal Chemists (AACC), (1983), Methods 22B60 8th Ed. St Paul, MN). Protein content was calculated by 6.25 X total N.

### EXAMPLES

The following examples are provided in order to demonstrate and further illustrate certain preferred embodiments and aspect of the present invention and are not to be construed as limiting the scope thereof.

### Example 1- Isolation and Cloning of the TrGA

Chromosomal DNA of *Trichoderma reesei* QM6a was isolated from mycelial mass of a liquid culture in Potato Dextrose Broth (Difco™ Cat. No. 254920) using the BIO101 Fast Prep^{®} System according to the method described by the supplier (Qbiogene). The DNA was purified using a Quick Spin column (Qiagen art No. 28106). The glucoamylase gene was isolated using primers with GA-specific sequences, NSP232R (SEQ ID NO: 24) and NSP233F (SEQ ID NO: 25) designed according to the predicted nucleotide sequence in the Trichoderma reesei genome database of Department of Energy (DOE) Joint Genome Institute. The primers were flanked at the 5'-end by Gateway^{®} attB sequences (Invitrogen). T. reesei QM6a chromosomal DNA was used as template.

The PCR mix contained the following components: Forward primer (10µM) 4µL; Reverse primer (10µM) 4µL; template DNA (500ng/µL) 1 µL; dNTPmix (10mM) 2µL; 10x Cx buffer 10µL, and PfuTurbo^{®} Cx Hotstart DNA polymerase 0.5µL (Stratagen Cat. No. 600410). Deionized water was added up to a total volume of 100µL.

The PCR protocol was as follows: Initial denaturation for 30 sec. at 98°C, denaturation , annealing and extension in 30 cycles of 10 sec at 98°C; 30 sec at 68°C; 45 sec at 72°C , respectively, and a final extension step of 10 min at 72°C.

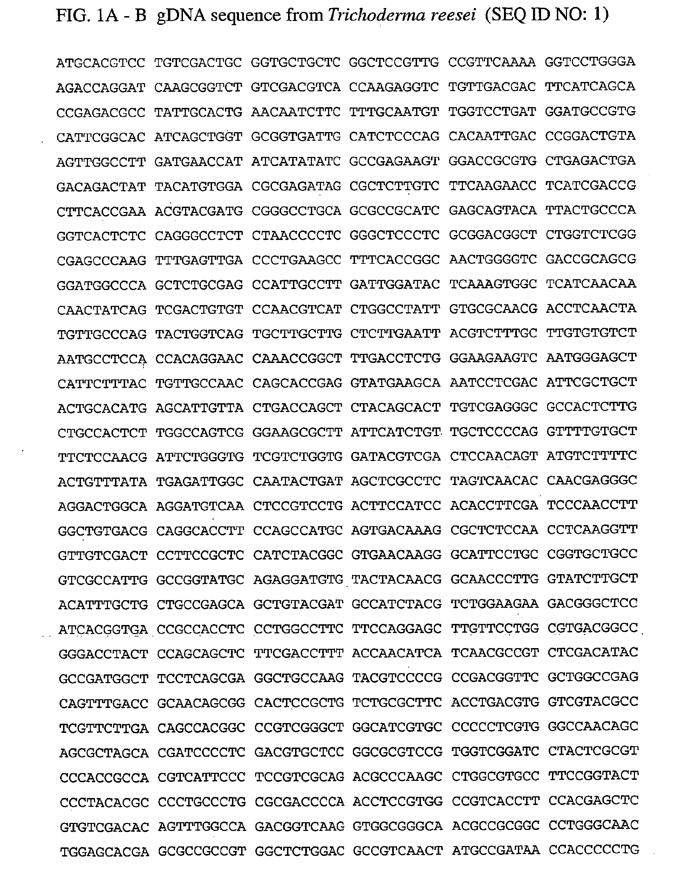

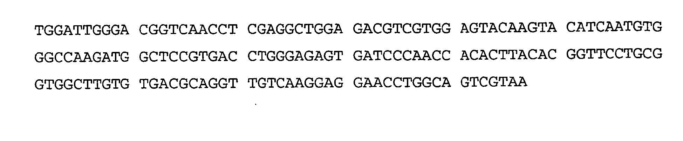

The PCR fragments were analyzed by electrophoresis in 1% agarose. Fragments of the expected size were isolated using the Gel-Extraction Purification Kit (Qiagene Cat. no. 28706). The PCR fragments were cloned into the Gateway ® Entry vector pDONR201 and transformed into E.coli DH5alpha Max Efficiency cells (Invitrogen Cat.No. 18258012). The nucleotide sequence of the inserted DNA was determined, from which the genomic DNA sequence of the TrGA gene was deduced (Fig. 1 (SEQ ID NO: 1)).

### Example 2 - Transformation of T. reesei and Fermentation/ Expression of the TrGA

Vector DNA containing the correct GA gene sequence was recombined into the *T. reesei* expression vector pTrex3g (Fig. 17).

The vector pTrex3g is based on the *E. coli* vector pSL1180 (Pharmacia Inc., Piscataway, NJ) which is a pUC118 phagemid based vector (Brosius, J. (1989), DNA 8:759) with an extended multiple cloning site containing 64 hexamer restriction enzyme recognition sequences: It was designed as a Gateway destination vector (Hartley et al., (2000) Genome Research 10:1788 -1795) to allow insertion using Gateway technology (Invitrogen) of any desired open reading frame between the promoter and terminator regions of the *T. reesei cbh*1 gene. It also contains the *Aspergillus nidulans amd*S gene for use as a selective marker in transformation of *T. reesei.* The details of the pTrex3g vector are as follows (Figure 17A). The vector is 10.3 kb in size. Inserted into the polylinker region of pSL1180 are the following segments of DNA: a) A 2.2 bp segment of DNA from the promoter region of the *T. reesei cbh*1 gene; b) the 1.7 kb Gateway reading frame A cassette acquired from Invitrogen that includes the attR1 and attR2 recombination sites at either end flanking the chloramphenicol resistance gene (CmR) and the ccdB gene; c) a 336 bp segment of DNA from the terminator region of the *T. reesei cbh*1 gene; and d) a 2.7kb fragment of DNA containing the *Aspergillus nidulans amd*S gene with its native promoter and terminator regions.

The expression vector containing the *T. reesei* GA gene, pNSP23 (Fig. 17) was transformed into a *T. reesei* host strain derived from RL-P37 (IA52) and having various gene deletions (Δcbh1, Δcbh2, Δea1, Δeg2) using particle bombardment by the PDS-1000/Helium System (BioRad Cat. No. 165-02257). The protocol is outlined below, and reference is also made to examples 6 and 11 of WO 05/001036.

A suspension of spores (approximately 5x10⁸ spores/ml) from a quad deleted strain of T. reesei was prepared. 100 ul- 200 ul of spore suspension was spread onto the center of plates of Minimal Medium (MM) acetamide medium. (MM acetamide medium had the following compositions: 0.6 g/L acetamide; 1.68 g/LCsCl; 20 g/L glucose; 20 g/L KH₂PO₄; 0.6 g/L CaCl₂ 2H₂O; 1 m1/L 1000X trace elements solution; 20 g/L Noble agar; and pH 5.5. 1000X trace elements solution contained 5.0 g/L FeSO₄ 7H₂O; 1.6 g/L MnSO₄; 1.4 g/L ZnSO₄ 7H₂O and 1.0 g/L CoCl₂ 6H₂O. The spore suspension was allowed to dry on the surface of the MM acetamide medium.

Transformation followed the manufacturers instruction. Briefly, 60 mg of M10 tungsten particles were placed in a microcentrifuge tube. 1 mL of ethanol was added and allowed to stand for 15 seconds. The particles were centrifuged at 15,000 rpm for 15 seconds. The ethanol was removed and the particles were washed three times with sterile dH₂O before 250 uL of 50% (v/v) sterile glycerol was added. 25 ul of tungsten particle suspension was placed into a microtrifuge tube. While continuously vortexing, the following were added: 5 ul (100 - 200 ng/ul) of plasmid DNA, 25 ul of 2.5M CaCl₂ and 10 ul of 0.1M spermidine. The particles were centrifuged for 3 seconds. The supernatant was removed and the particles were washed with 200 ul of 100% ethanol and centrifuged for 3 seconds. The supernatant was removed, 24 uL 100% ethanol was added and mixed by pipetting, then 8 ul aliquots of particles were removed and placed onto the center of macrocarrier disks that were held in a desiccator. Once the tungsten/DNA solution had dried the macrocarrier disk was placed in the bombardment chamber along with the plate of MM acetamide with spores and the bombardment process was performed according to the manufacturers instructions. After bombardment of the plated spores with the tungsten/DNA particles, the plates were incubated at 30°C. Transformed colonies were transferred to fresh plates of MM acetamide medium and incubated at 30°C.

### Example 3 - Demonstration of GA activity from the expressed TrGA in transformed cells

After 5 days of growth on MM acetamide plates transformants displaying stable morphology were inoculated into 250 ml shake flasks containing 30 ml of Proflo medium. (Proflo medium contained: 30 g/L α-lactose; 6.5 g/L (NH₄)₂SO₄; 2 g/L KH₂PO₄; 0.3 g/L MgSO₄ 7H₂O 0.2 g/L CaCl₂; 1 ml/L 1000X trace element salt solution; 2 ml/L 10% Tween 80; 22.5 g/L ProFlo cottonseed flour (Traders protein, Memphis, TN); 0.72 g/L CaCO₃ After two days growth at 28 C and 140rpm, 10% of the Proflo culture was transferred to a 250 ml shake flask containing 30 ml of Lactose Defined Media. The composition of the Lactose defined Media was as follows 5 g/L (NH₄)₂SO₄; 33 g/L PIPPS buffers; 9 g/L casamino acids; 4.5 g/L KH₂PO₄; 1.0 g/L MgSO₄ 7H₂O; 5 ml/L Mazu DF60-P antifoam (Mazur Chemicals, IL); 1000X trace element solution; pH 5.5; 40 ml/L of 40% (w/v) lactose solution was added to the medium after sterilization. The Lactose Defined medium shake flasks were incubated at 28°C, 140 rpm for 4 - 5 days.

Mycelium was removed by centrifugation and the supernatant was analyzed for total protein (BCA Protein Assay Kit, Pierce Cat. No. 23225) and GA activity using pNPG as substrate (Sigma N-1377).

Samples of the culture supernatant were mixed with appropriate volume of 2X sample laoding buffer with reducing agent and the protein profile was determined by sodium dodecyl sulfate-polyacrylamide gel electrophoresis (SDS-PAGE) using NuPAGE^{®} Novex 10% Bis-Tris Gel with MES SDS Running Buffer. The gels were stained with SimplyBlueTm SafeStain (Invitrogen, Carlsbad, CA, USA).

On SDS-PAGE analysis a protein band that was not observed in supernatant from a quad delete strain was observed in the supernatant of some transformants with the pTrex3g vector containing the glucoamylase open reading frame (Figure 16). This new protein band had an apparent molecular weight of approximately 64 kDa. This result confirms that TrGA is secreted into the medium.

### Example 4 - Biochemical characterization of the GA gene product

GA producing transformants were grown at 4-L scale. The culture filtrate was concentrated using an ultra filtration unit with a nominal molecular weight limit of 10,000 Da (Pall Omega Centramate OS010c10). The crude enzyme preparation was purified by a 2-step procedure using an ÄKTA explorer 100 FPLC System (Amersham Biosciences). A HiPrep 16/10 FF Q-Sepharose column (Amersham BioSciences Cat. No. 17-5190-01) was equilibrated with 25 mM Tris pH 8.0 and the protein was eluted from the column with 100 mM NaCl in 25 mM Tris pH 8.0. A second affinity chromatography step was performed using Cbind 200 resin (Novagen Cat. No. 701212-3) and 50 mM Tris pH 7.0 containing 500 mM NaCl as elution buffer (Fig. 16). The N-terminus of the gene product (Ser-Val-Asp-Asp-Phe-Ile) (SEQ ID NO: 38) was determined by Edman degradation (Edman, P. (1956) Acta Chem Scand 10:761-768).

The pH and temperature profiles of the glucoamylase activity of the gene product were determined using 4-nitrophenyl-α-D-glucopyranoside as substrate (Elder, M. T. and Montgomery R. S., Glucoamylase activity in industrial enzyme preparations using colorimetric enzymatic method; Collaborative study Journal of AOAC International, vol. 78(2), 1995) (Fig. 18).

### Example 5 - Isolation cloning of glucoamylase homologs from strains in the Trichoderma/Hypocrea family cluster

Chromosomal DNA preparations of the strains (GA102) *-Hypocrea citrina* var. *americana* (CBS976.69); (GA104) *- Hypocrea vinosa* (CBS960.68); (GA105)-*Trichoderma sp; (GA107) - Hypocrea gelatinosa* (CBS254.62); GA108 - *Hypocrea orientalis* (ATCC 90550); (GA109) - *Trichoderma konilangbra;* (GA103) - *Trichoderma harzianum* (CBS433.95); (GA113) - *Trichoderma sp.;* (GA124) - *Trichoderma longibrachiatum;* (GA127) - *Trichoderma asperellum* (ATCC 28020); and (GA128) - *Trichoderma strictipilis* (CBS 347.93) were isolated as described in example 1. Full-length GA genes were cloned as described in example 1 using the TrGA-gene specific primers NSP231F (SEQ ID NO: 23) and NSP232R (SEQ ID NO: 24). The nucleotide sequences of the strains are disclosed in Fig. 4 for GA102 (SEQ ID NO: 5); Fig. 5 for GA104 (SEQ ID NO: 7); Fig. 6 for GA105 (SEQ ID NO: 9); Fig. 7 for GA107 (SEQ ID NO: 11); Fig. 8 for GA108 (SEQ ID NO: 13); Fig. 9 for GA109 (SEQ ID NO: 15); Fig. 10 for GA113 (SEQ ID NO: 28); Fig. 11 for GA103 (SEQ ID NO: 30); Fig. 12 for GA124 (SEQ ID NO: 32); Fig. 13 for GA127 (SEQ ID NO: 34) and Fig. 14 for GA128 (SEQ ID NO: 36). The corresponding amino acid sequences are illustrated in Fig. 15. Table 2 sets forth the percent identity of the amino acid sequences of the mature protein of *T. reesei* glucoamylase (Fig. 3B, SEQ ID NO: 4) with the glucoamylase homologs from the HypocrealTrichoderma cluster.

**Table 2 - % Identity of GA homologs from the Hypocrea/Trichoderma cluster**

| | GA 102 | GA 103 | GA 104 | GA 105 | GA 107 | GA 108 | GA 109 | GA 113 | GA 124 | GA 127 | GA 128 | TrGA |
|---|---|---|---|---|---|---|---|---|---|---|---|---|
| GA102 | 100 | 86 | 86 | 84 | 87 | 84 | 84 | 83 | 84 | 87 | 85 | 84 |
| GA103 | | 100 | 98 | 90 | 96 | 90 | 91 | 86 | 90 | 98 | 90 | 90 |
| GA104 | | | 100 | 91 | 97 | 91 | 90 | 86 | 91 | 99 | 90 | 91 |
| GA105 | | | | 100 | 90 | 95 | 93 | 83 | 94 | 91 | 94 | 95 |
| GA107 | | | | | 100 | 90 | 90 | 86 | 90 | 98 | 90 | 90 |
| GA108 | | | | | | 100 | 94 | 84 | 98 | 91 | 94 | 97 |
| GA109 | | | | | | | 100 | 83 | 94 | 91 | 94 | 94 |
| GA113 | | | | | | | | 100 | 84 | 86 | 83 | 84 |
| GA124 | | | | | | | | | 100 | 91 | 94 | 98 |
| GA127 | | | | | | | | | | 100 | 91 | 91 |
| GA128 | | | | | | | | | | | 100 | 94 |
| TrGA | | | | | | | | | | | | 100 |

*T. reesei* strains over-expressing GA were obtained as described in example 2. Crude enzyme preparations were obtained as described in example 3 and Fig. 19 illustrates the gels obtained for some of the homologs. Table 3 sets forth the glucoamylase activity of some of the homologs.

**Table 3**

| Strain | Gene from: | Total protein mg/mL | U GA/mL | Specific Activity |
|---|---|---|---|---|
| GA104 | *H. vinosa* | 2.76 | 37 | 13 |
| GA105 | *T. sp.* | 2.77 | 26 | 9 |
| GA107 | *H. gelatinosa* | 3.61 | 178 | 49 |
| GA109 | *T. konilangbra* | 2.22 | 10 | 5 |
| TrGA | *T. reesei* | 3.89 | 91 | 23 |
| Host Control | *T. reesei* | 0.7 | 3 | 4 |

### Example 6 - Glucose Production Using TrGA

A 32% DS slurry of Cargill bag starch was made up with reverse osmosis water. The pH of the slurry was adjusted to pH 5.8. The slurry was filtered through a 100-mesh screen and dosed at 4.0 AAU/g ds using SPEZYME® ETHYL, (Genencor International, Inc.). The slurry was then jetted at 107.3°C for 5 min (primary liquefaction). Enzyme activity is determined by the rate of starch hydrolysis, as reflected in the rate of decrease in iodine-staining capacity. One AAU unit of bacterial alpha amylase activity is the amount of enzyme required to hydrolyze 10 mg of starch per minute under specified conditions. After primary liquefaction, the liquefact was collected and placed in a 95°C water bath for 120 min (Secondary liquefaction). Samples were taken at 30, 60, 90 and 120 min and checked for DE by using the standard Schoorls reducing sugar method from the scorn Refiners Association. The liquefact was aliquoted in 100-g quantities into screw cap jars, the pH was adjusted to pH 4.5 and equilibrated to 60°C for 15 minutes prior to dosing. The TrGA enzyme was diluted so as to add 0.2 mls of diluted enzyme to the jar at 0.22 GAU/g ds. After dosing, the liquefact was aliquoted into 7 screw cap tubes, each containing approximately 10 mls of material and returned to the designated temperature. Tubes were removed at selected time intervals (18, 24, 30, 42, 50 and 55 hours) and analyzed by HPLC Carbohydrate System: Column: Phenomenex Rezex Carbohydrate (RCM- Monosaccharide) #00H-0130-KO (Equivalent to Bio-Rad 87H); Column Temperature: 70°C; Mobile Phase: Nanopure DI H₂O Flow Rate: 0.8 mL/min; Detector: RI; Injection Volumn: 10µL (3% DS material) for sugar composition.

**Table 4- Production of Glucose from cornstarch using TrGA**

| Treatment (hrs) | %DP1 | %DP2 | %DP3 | %DP > 3 |
|---|---|---|---|---|
| 18 | 80.66 | 2.60 | 0.66 | 16.08 |
| 24 | 84.25 | 2.31 | 0.46 | 12.98 |
| 30 | 86.26 | 2.66 | 0.47 | 10.61 |
| 42 | 88.85 | 3.05 | 0.40 | 7.69 |
| 50 | 89.93 | 3.26 | 0.42 | 6.39 |
| 55 | 90.64 | 3.36 | 0.37 | 5.62 |

### Example 7 - Ethanol Production using TrGA in a Simultaneous Saccharification and Fermentation (SSF) Process

A sample of corn mash liquefact from a local ethanol producer was obtained and diluted to 29% DS using thin stillage. The pH of the slurry was adjusted to pH 4.3 using 6 N sulphuric acid. A 300 g aliquot of the mash was placed into a 31°C water bath and allowed to equilibrate. TrGA was added to the sample (0.4 GAU/g ds, which is equal to 1.08 kg/MT ds). After enzyme addition, 1 ml of a 15 g in 45 ml DI water solution of Red Star Red yeast (Lesaffre yeast Corp. Milwaukee, WI) was added to each sample. Samples were taken at 18, 26, 41 and 53 hours and analyzed by HPLC Column: Phenomenex Rezex organic Acid Column (RHM- Monosaccharide) #00H-0132-KO (Equivalent to Bio-Rad 87H); Column Temperature: 60°C; Mobile Phase: 0.01 NH₂SO₄; Flow Rate: 0.6 mL/min; Detector: RI; Injection Volumn:20µL.

**Table 5 - Production of Ethanol by TrGA (0.4 GAU/g) in a SSF Process**

| Sample (hrs) | % w/v DP >3 | % w/v DP-3 | % w/v DP-2 | % w/v DP-1 | % w/v Lactic Acid | % w/v Glycerol | % v/v EtOH |
|---|---|---|---|---|---|---|---|
| 18 | 6.38 | 0.61 | 3.42 | 2.69 | 0.31 | 0.97 | 7.44 |
| 26 | 4.39 | 0.76 | 1.02 | 1.81 | 0.30 | 1.12 | 10.68 |
| 41 | 1.62 | 0.47 | 0.35 | 0.77 | 0.31 | 1.27 | 13.65 |
| 53 | 1.03 | 0.37 | 0.36 | 0.16 | 0.32 | 1.32 | 14.46 |

### Example 8 - A Non-cook Process for Ethanol Production using TrGA.

In general a 33% slurry of corn flour (Azure Standard Farms) was prepared in DI H₂O to which 400 ppm urea was added. The pH was adjusted to 4.5. Fermentations were conducted in 125 ml flasks containing 100g mash and various treatments of GAU/g TrGA. A 20% slurry of Fali dry yeast in water was prepared and mixed with a 32°C water bath one hour prior to inoculating the fermenters by adding 0.2 ml of the yeast slurry. The flasks were placed in a 32 °C water bath and the mash mix gently. During the fermentations samples were removed for HPLC analysis. The fermentations were terminated after 72 hours. Production of compounds including sugars, lactic acid, glycerol and ethanol at various sampling intervals is shown below in various tables. The mash was dried at 60°C to obtain the DDGS, and the starch content of the DDGS was determined by the dual enzyme method.

### A. All conditions were as described above: the treatment included 1.2 GAU/g TrGA.

**Table 6 - Ethanol Production**

| Treatment | Hrs | %W/V DP>3 | %W/V DP-3 | %W/V DP-2 | %W/V DP-1 | %W/V Lactic | %W/V Glycerol | %V/V EtOH |
|---|---|---|---|---|---|---|---|---|
| TrGA | 17 | 0.68 | 0.05 | 0.04 | 0.00 | 0.04 | 0.41 | 4.70 |
| TrGA | 24 | 0.67 | 0.06 | 0.05 | 0.02 | 0.04 | 0.42 | 5.44 |
| TrGA | 41 | 0.65 | 0.07 | 0.00 | 0.00 | 0.05 | 0.44 | 6.78 |
| TrGA | 48 | 0.59 | 0.08 | 0.08 | 0.00 | 0.07 | 0.43 | 7.77 |
| TrGA | 64 | 0.61 | 0.08 | 0.00 | 0.00 | 0.15 | 0.43 | 8.42 |
| TrGA | 72 | 0.60 | 0.08 | 0.07 | 0.01 | 0.17 | 0.43 | 8.59 |

### B. All conditions were as described above: the treatments included 0.75 GAU/g GA107 and 0.75GAU/g GA104

**Table 7 - Ethanol Production**

| GA | hrs | %w/v DP>3 | %w/v DP-3 | %w/v DP-2 | % w/v DP-1 | % w/v Lactic | % w/v Glycerol | % v/v ETOH |
|---|---|---|---|---|---|---|---|---|
| | | 1.11 | 0.10 | 0.29 | 1.06 | 0.00 | 0.15 | 0.00 |
| 104 | 13 | 0.84 | 0.00 | 0.01 | 0.01 | 0.00 | 0.43 | 5.03 |
| 107 | 13 | 0.77 | 0.00 | 0.00 | 0.00 | 0.00 | 0.42 | 4.16 |
| 104 | 21 | 0.94 | 0.14 | 0.03 | 0.00 | 0.00 | 0.46 | 6.90 |
| 107 | 21 | 0.88 | 0.10 | 0.03 | 0.01 | 0.00 | 0.43 | 5.24 |
| 104 | 35 | 0.94 | 0.18 | 0.13 | 0.02 | 0.02 | 0.49 | 9.02 |
| 107 | 35 | 0.87 | 0.11 | 0.02 | 0.01 | 0.04 | 0.44 | 6.53 |
| 104 | 54 | 0.91 | 0.14 | 0.00 | 0.00 | 0.00 | 0.51 | 10.93 |
| 107 | 54 | 0.89 | 0.13 | 0.00 | 0.00 | 0.30 | 0.45 | 7.58 |
| 104 | 62 | 0.87 | 0.12 | 0.00 | 0.00 | 0.00 | 0.53 | 11.49 |
| 107 | 62 | 0.88 | 0.14 | 0.00 | 0.00 | 0.39 | 0.46 | 7.74 |
| 104 | 72 | 0.94 | 0.14 | 0.16 | 0.00 | 0.00 | 0.53 | 12.22 |
| 107 | 72 | 0.88 | 0.14 | 0.05 | 0.01 | 0.42 | 0.47 | 7.82 |

### C. All conditions were as described above: the treatments included a) A. niger GA 0.75 GAU/g + 2.25 SSU AkAA and b) TrGA 0.75 GAU/g + 2.25 SSU AkAA. The residual starch for AnGA + AkAA treatment was determined to be 5.26% and the residual starch for TrGA + AkAA treatment was determined to be 8.71%.

The measurement of alpha amylase activity for AkAA is based on the degree of hydrolysis of soluble potato starch substrate (4% ds) by an aliquot of the enzyme sample at pH 4.5, 50°C. The reducing sugar content is measured using the DNS method as described in Miller, G. L. (1959) Anal. Chem. 31:426 - 428. One unit of the enzyme activity (SSU; soluble starch unit) is equivalent to the reducing power of 1mg of glucose released per minute at the specific incubation conditions.

**Table 8 - Ethanol Production**

| Treatment | Hours | %W/V DP>3 | %W/V DP-3 | %W/V DP-2 | %W/V DP-1 | %W/V Lactic | %W/V Glycerol | %V/V Ethanol |
|---|---|---|---|---|---|---|---|---|
| AnGA + AkAA | 15 | 0.81 | 0.00 | 0.04 | 0.13 | 0.04 | 0.63 | 8.22 |
| TrGA + AkAA | 15 | 0.94 | 0.00 | 0.04 | 0.03 | 0.04 | 0.68 | 8.35 |
| | | | | | | | | |
| AnGA + AkAA | 26.5 | 0.94 | 0.06 | 0.04 | 0.08 | 0.06 | 0.89 | 12.59 |
| TrGA + AkAA | 26.5 | 1.00 | 0.08 | 0.08 | 0.00 | 0.06 | 0.83 | 11.81 |
| | | | | | | | | |
| AnGA + AkAA | 40 | 0.65 | 0.10 | 0.08 | 0.05 | 0.06 | 0.94 | 14.37 |
| TrGA + AkAA | 40 | 0.73 | 0.10 | 0.14 | 0.00 | 0.05 | 0.91 | 13.80 |
| | | | | | | | | |
| AnGA + AkAA | 49 | 0.93 | 0.07 | 0.06 | 0.05 | 0.05 | 1.08 | 17.05 |
| TrGA + AkAA | 49 | 0.98 | 0.08 | 0.14 | 0.00 | 0.04 | 0.97 | 15.52 |
| | | | | | | | | |
| AnGA + AkAA | 70 | 0.82 | 0.04 | 0.04 | 0.27 | 0.00 | 1.07 | 17.59 |
| TrGA + AkAA | 70 | 0.95 | 0.08 | 0.04 | 0.00 | 0.00 | 1.01 | 17.17 |

### D. All conditions were as described above: the treatments included a) TrGA 0.695 GAU/g + 2.25 SSU AkAA and b) TrGA 0.695 GAU/g + 2.25 SSU AKAA + 2 ASPU/g Pullulanase. One acid stable pullulanase unit (ASPU) is defined as the amount of enzyme which liberates one equivalent reducing potential as glucose per minute from pullulan at pH 4.5 and a temperature of 60°C.

**Table 9 - Ethanol production**

| | | % WN | % W/V | % W/V | % W/V | % W/V | % W/V | % V/V | DDGS % |
|---|---|---|---|---|---|---|---|---|---|
| Treatment | Hours | DP>3 | DP-3 | DP-2 | DP-1 | Lactic | Glycerol | Ethanol | starch |
| | | | | | | | | | |
| TrGA | 15 | 0.92 | 0.05 | 0.05 | 0.04 | 0.03 | 0.60 | 7.69 | |
| TrGA + Pullulanase | 15 | 0.91 | 0.05 | 0.04 | 0.04 | 0.03 | 0.60 | 8.00 | |
| | | | | | | | | | |
| TrGA | 24 | 0.94 | 0.08 | 0.09 | 0.05 | 0.04 | 0.72 | 10.46 | |
| TrGA + Pullulanase | 24 | 0.91 | 0.12 | 0.10 | 0.05 | 0.04 | 0.73 | 10.93 | |
| | | | | | | | | | |
| TrGA | 41 | 0.91 | 0.10 | 0.17 | 0.05 | 0.04 | 0.86 | 13.89 | |
| TrGA + Pullulanase | 41 | 0.92 | 0.13 | 0.16 | 0.04 | 0.05 | 0.87 | 14.33 | |
| | | | | | | | | | |
| TrGA | 47 | 0.87 | 0.10 | 0.20 | 0.05 | 0.04 | 0.90 | 14.51 | |
| TrGA + Pullulanase | 47 | 0.94 | 0.13 | 0.19 | 0.04 | 0.03 | 0.91 | 15.32 | |
| | | | | | | | | | |
| TrGA | 70 | 0.92 | 0.11 | 0.06 | 0.03 | 0.00 | 0.98 | 17.27 | 18.5 |
| TrGA + Pullulanase | 70 | 0.95 | 0.11 | 0.05 | 0.02 | 0.00 | 0.98 | 17.77 | 16.4 |

### E. All conditions were as described above: the treatments included TrGA 0.695 GAU/g and the following AkAA treatments: a) 3 SSU AkAA; b) 10 SSU AkAA and c) 30 SSU AkAA.

**Table 10**

| Treatment (AkAA) | Hours | %w/v DP>3 | %w/v DP-3 | %w/v DP-2 | %w/v DP-1 | %w/v Lactic | %w/v Glycerol | % v/v Ethanol | % starch DDGS |
|---|---|---|---|---|---|---|---|---|---|
| 3 SSU | 17 | 0.77 | 0.05 | 0.00 | 0.00 | 0.04 | 0.59 | 8.31 | |
| 10 SSU | 17 | 0.76 | 0.04 | 0.03 | 0.00 | 0.03 | 0.62 | 8.85 | |
| 30 SSU | 17 | 0.78 | 0.04 | 0.05 | 0.00 | 0.03 | 0.06 | 9.54 | |
| | | | | | | | | | |
| 3 SSU | 30 | 0.74 | 0.07 | 0.05 | 0.00 | 0.05 | 0.73 | 11.35 | |
| 10 SSU | 30 | 0.78 | 0.06 | 0.05 | 0.00 | 0.05 | 0.81 | 12.62 | |
| 30 SSU | 30 | 0.85 | 0.71 | 0.05 | 0.03 | 0.05 | 0.84 | 13.91 | |
| | | | | | | | | | |
| 3 SSU | 41 | 0.70 | 0.08 | 0.02 | 0.02 | 0.05 | 0.90 | 13.96 | |
| 10 SSU | 41 | 0.69 | 0.08 | 0.02 | 0.03 | 0.05 | 0.91 | 15.02 | |
| 30 SSU | 41 | 0.68 | 0.07 | 0.03 | 0.07 | 0.05 | 0.92 | 15.83 | |
| | | | | | | | | | |
| 3 SSU | 51 | 0.73 | 0.09 | 0.09 | 0.04 | 0.05 | 0.98 | 15.38 | |
| 10 SSU | 51 | 0.74 | 0.09 | 0.05 | 0.05 | 0.04 | 0.99 | 16.57 | |
| 30 SSU | 51 | 0.73 | 0.08 | 0.04 | 0.03 | 0.04 | 0.96 | 16.53 | |
| | | | | | | | | | |
| 3 SSU | 70 | 0.70 | 0.09 | 0.02 | 0.02 | 0.02 | 1.04 | 17.09 | 15.8 |
| 10 SSU | 70 | 0.72 | 0.08 | 0.02 | 0.03 | 0.04 | 1.04 | 17.35 | 10.7 |
| 30 SSU | 70 | 0.71 | 0.08 | 0.02 | 0.07 | 0.03 | 1.01 | 17.42 | 9.6 |

## Claims

1. An enzyme composition comprising a glucoamylase having an amino acid sequence that is at least 90% identical to SEQ ID NO: 4 and an alpha amylase from *Aspergillus,* wherein the alpha amylase has at least 90% identity to the mature protein sequence of SEQ ID NO: 27, and wherein the specific activity of said glucoamylase is at least 90% of the specific activity of a glucoamylase having the sequence of SEQ ID NO: 4.

2. An enzyme composition according to claim 1 wherein the glucoamylase has at least 95% or 97% sequence identity to SEQ ID NO: 4.

3. An enzyme composition according to claim 1 wherein the alpha amylase has at least 93%, 95%, 96%, 97%, 98% or 99% identity to the mature protein sequence of SEQ ID NO: 27.

4. A method of hydrolyzing starch comprising treating a starch-containing substrate with an enzyme composition according to any one of claims 1 to 3.

5. A method for producing a fermentation product from a granular starch containing substrate comprising
a) contacting a granular starch containing substrate with an enzyme composition according to any one of claims 1 to 3 at a temperature below the gelatinization temperature, at a pH of about 4 to 7.0 for a period of time to produce a composition comprising glucose, and
b) contacting the glucose with a fermentation organism under suitable fermentation conditions to produce a fermentation product.

6. The method of claim 5, wherein the fermentation product is ethanol.

7. A method for saccharifying liquefied starch comprising treating liquefied starch with an enzyme composition according to any one of claims 1 to 3 and obtaining a composition which includes at least 80% glucose.

## Patentansprüche

1. Enzymzusammensetzung, umfassend eine Glucoamylase mit einer Aminosäuresequenz, die zumindest zu 90 % mit Seq.-ID Nr. 4 identisch ist, und eine α-Amylase aus Aspergillus, worin die α-Amylase zumindest 90 % Identität mit der reifen Proteinsequenz von Seq.-ID Nr. 27 aufweist und worin die spezifische Aktivität der Glucoamylase zumindest 90 % der spezifischen Aktivität einer Glucoamylase mit der Sequenz von Seq.-ID Nr. 4 beträgt.

2. Enzymzusammensetzung nach Anspruch 1, worin die Glucoamylase zumindest 95 % oder 97 % Sequenzidentität mit Seq.-ID Nr. 4 aufweist.

3. Enzymzusammensetzung nach Anspruch 1, worin die α-Amylase zumindest 93 %, 95 %, 97 %, 98 % oder 99 % Identität mit der reifen Proteinsequenz von Seq.-ID Nr. 27 aufweist.

4. Verfahren zum Hydrolysieren von Stärke, umfassend das Versetzen eines stärkehältigen Substrats mit einer Enzymzusammensetzung nach einem der Ansprüche 1 bis 3.

5. Verfahren zur Herstellung eines Fermentationsprodukts aus einem Substrat, das körnige Stärke enthält, umfassend:
a) das Kontaktieren eines Substrats, das körnige Stärke enthält, mit einer Enzymzusammensetzung nach einem der Ansprüche 1 bis 3 bei einer Temperatur unterhalb der Gelierungstemperatur, bei einem pH-Wert von etwa 4 bis 7,0 und für einen bestimmten Zeitraum, um eine Glucose umfassende Zusammensetzung zu bilden, und
b) das Kontaktieren der Glucose mit einem Fermentationsorganismus unter geeigneten Fermentationsbedingungen, um ein Fermentationsprodukt zu produzieren.

6. Verfahren nach Anspruch 5, worin das Fermentationsprodukt Ethanol ist.

7. Verfahren zum Verzuckern verflüssigter Stärke, umfassend das Versetzen verflüssigter Stärke mit einer Enzymzusammensetzung nach einem der Ansprüche 1 bis 3 und das Erhalten einer Zusammensetzung, die zumindest 80 % Glucose umfasst.

## Revendications

1. Composition d'enzymes comprenant une glucoamylase ayant une séquence d'acides aminés qui est au moins à 90% identique avec la SEQ ID NO : 4 et une alpha amylase de *Aspergillus*, où l'alpha amylase possède au moins 90% d'identité avec la séquence de protéines matures de la SEQ ID NO: 27, et où l'activité spécifique de ladite glucoamylase représente au moins 90% de l'activité spécifique d'une glucoamylase ayant la séquence de SEQ ID NO : 4.

2. Composition d'enzymes selon la revendication 1, où la glucoamylase a au moins 95% ou 97% d'identité de séquence avec la SEQ ID NO : 4.

3. Composition d'enzymes selon la revendication 1, où l'alpha amylase a au moins 93%, 95%, 96%, 97%, 98% ou 99% d'identité avec la séquence de protéines matures de la SEQ ID NO : 27.

4. Méthode d'hydrolysation d'un amidon comprenant le traitement d'un substrat contenant de l'amidon avec une composition d'enzymes selon l'une quelconque des revendications 1 à 3.

5. Méthode de production d'un produit de fermentation à partir d'un substrat contenant un amidon granulaire comprenant
a) mettre en contact un substrat contenant de l'amidon granulaire avec une composition d'enzymes selon l'une quelconque des revendications 1 à 3 à une température en dessous de la température de gélatinisation, à un pH d'environ 4 à 7,0 pendant une période de temps pour produire une composition comprenant du glucose, et
b) mettre en contact le glucose avec un organisme de fermentation sous des conditions de fermentation appropriées pour produire un produit de fermentation.

6. Méthode selon la revendication 5, où le produit de fermentation est l'éthanol.

7. Méthode de saccharification d'amidon liquéfié comprenant le traitement de l'amidon liquéfié avec une composition d'enzymes selon l'une quelconque des revendications 1 à 3 et l'obtention d'une composition qui comprend au moins 80% de glucose.
